(19) **Europäisches Patentamt / European Patent Office / Office européen des brevets**

(11) **EP 4 064 975 B1**

(12) **EUROPEAN PATENT SPECIFICATION**

(45) Date of publication and mention of the grant of the patent:
**10.09.2025 Bulletin 2025/37**

(21) Application number: **20821097.1**

(22) Date of filing: **27.11.2020**

(51) International Patent Classification (IPC):
**A61B 5/021** (2006.01)   **A61B 5/00** (2006.01)
**A61B 5/0215** (2006.01)

(52) Cooperative Patent Classification (CPC):
**A61B 5/021; A61B 5/02108; A61B 5/0215; A61B 5/4836**

(86) International application number:
**PCT/EP2020/083762**

(87) International publication number:
**WO 2021/105448 (03.06.2021 Gazette 2021/22)**

(54) **CARDIOVASCULAR ANALYTIC SYSTEM AND METHOD**

KARDIOVASKULÄRES ANALYTISCHES SYSTEM UND VERFAHREN

SYSTÈME ET PROCÉDÉ ANALYTIQUE CARDIOVASCULAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.11.2019 GB 201917286**

(43) Date of publication of application:
**05.10.2022 Bulletin 2022/40**

(73) Proprietor: **Directed Systems Limited**
**Cambridge, Cambridgeshire CB4 0WS (GB)**

(72) Inventors:
• **LEANING, Mark Stephen**
**Cambridge CB5 8HX (GB)**
• **LEANING, Philip Michael**
**Cambridge CB1 2PS (GB)**

(74) Representative: **Marks & Clerk LLP**
**15 Fetter Lane**
**London EC4A 1BW (GB)**

(56) References cited:
**EP-A1- 2 563 213      EP-B1- 2 563 213**
**WO-A1-2011/080185   WO-A1-2011/080190**
**WO-A2-2007/141246   CN-A- 108 378 835**

• **WILLIAM GEOFFREY PARKIN ET AL: "Therapeutic control of the circulation", JOURNAL OF CLINICAL MONITORING AND COMPUTING, KLUWER ACADEMIC PUBLISHERS, DO, vol. 22, no. 6, 12 November 2008 (2008-11-12), pages 391 - 400, XP019680686, ISSN: 1573-2614**

**Description**

FIELD OF THE INVENTION

The present invention relates to a cardiovascular analytic system. In particular, the present invention relates to a system for determining relative changes in at least one parameter of cardiovascular function.

BACKGROUND OF THE INVENTION

Episodes of low blood pressure (hypotension) and associated complications occur in all areas of critical care where the patient's normal homeostasis is disturbed.

The problem has been most extensively researched and understood during surgical procedures where the patient is undergoing anaesthesia.

There are over 312 million surgical procedures performed annually globally (Weiser et al., 2008) (Ghaferi, Birkmeyer and Dimick, 2009). Whilst the majority of patients fall into a low risk group, it has been previously estimated that up to 15% of patients can be classified as high risk (Pearse et al., 2006). The concept of risk is important to understand: it defines a probability of an event (Cecconi, Corredor, et al., 2013) which, for high risk surgical patients is either mortality or a post-operative complication. This 15% of high risk patients (more than 45 million per year) account for 80% of the whole post-operative mortality (Pearse et al., 2006).

Importantly, if a patient survives the first post-operative days and after develops an in-hospital complication, he/she is more likely to experience a longer length of stay compared to a patient without complications. This is reflected into an increased expenditure (Ghaferi, Birkmeyer and Dimick, 2009) (Khuri et al., 2005) (Dimick et al., 2004). A patient with a major post-operative complication can generate costs 12 times higher than one without any. In practice a $5,000 operation can cost to the system up to $60,000 (Hamilton, Cecconi and Rhodes, 2011).

The problem does not stop in hospital. Patients who develop post-operative complications have a high risk of long-term morbidity and mortality. For instance, Khuri et al (Khuri et al., 2005) showed that patients who developed a post-operative complication within the first 30 days after surgery have a 30-day mortality of 13.3%, compared to 0.8% in those without post-operative complications. The effect persisted when comparing 1 and 5-year mortality between the two groups. Even more interestingly, the occurrence of post-operative complications was more important than any pre-operative risk factors in determining long term outcomes (Khuri et al., 2005). It is clear then why healthcare systems are working to improve the care of surgical patients to minimise the occurrence of post-operative complications.

Different strategies have been studied and adopted in different countries. These strategies go from goal directed therapy to enhanced recover after surgery (Cecconi, Corredor, et al., 2013) (Hamilton, Cecconi and Rhodes, 2011) (Fearon et al., 2005; Arulkumaran et al., 2014; Ebm et al., 2014; Scott et al., 2015). Usually these quality improvement initiatives add a little extra cost per patient but are cost-effective (and often cost-saving) since their implementation in the right populations can significantly reduce complications and length of stay (Ebm et al., 2014).

Reducing postoperative morbidity is a dynamic field of research and clinical practice. Patient selection, surgical and anaesthetic techniques have significantly improved over the years and it is possible that what worked years ago may not show the same benefit now. For this reason, it's important to carry on exploring new areas of improvement.

Recently a focus on basic physiological parameters has re-emerged with more and more data highlighting the importance of arterial blood pressure values during surgery (McCormick et al., 2016) (Walsh et al., 2013).

While it may seem intuitive that maintaining a good blood pressure during the peri-operative period is important for patients, a growing body of evidence shows that this does not happen and that intra-operative blood pressure values are extremely variable (Walsh et al., 2013; Hsieh et al., 2016; Salmasi et al., 2017). There are several reasons for this. The anaesthetic drugs can impair blood pressure control by altering the vasoactive tone of the vessels, impairing cardiac function and affecting the automatic control systems of the body. Surgical stress and bleeding are other important factors that can significantly affect blood pressure control.

Surprisingly, while there has been a significant amount of research on goal directed therapy (a technique that allows clinician to titrate fluids and vasoactive drugs toward optimisation of cardiac output) little research has focused on how to best optimise blood pressure intra- and post-operatively.

This is particularly worrying if one looks at the very convincing data showing that, even just a few minutes of mean arterial blood pressure below 55 mmHg, are associated with significant events in the postoperative period. These events go from myocardial infarctions to acute kidney injury and death (Walsh et al., 2013; Hirsch et al., 2015; Hsieh et al., 2016; Salmasi et al., 2017).

The lower the blood pressure, the less time is associated with an increased risk of complications. Stapelfeldt et al have estimated the risk associated with each mmHg level below 80 mmHg with an increased risk of complications and combined these into an overall score, the SLU Score, which is associated with post-operative mortality (Stapelfeldt et al., 2017).

In practice, whatever the reason is for a drop in blood pressure, the fact remains that dangerously low levels should be avoided and acted upon very fast if they occur.

Blood pressure variability is also important; recent data shows that for instance this variability is associated to postoperative delirium (Hirsch et al., 2015), another cause of prolonged length of stay.

Most research has focused on the association between intra-operative hypotension and the occurrence of post-operative complications. However, evidence is building that preventing hypotension leads to a reduction in complications (Futier et al., 2017).

It is unlikely that an educational and awareness campaign alone with anaesthesia providers can completely abolish these hypotensive episodes. It is more likely that this could be achieved via a technology that could alert the clinician of downwards trends and ideally intervene in case the human reaction is too slow.

As with intra-operative care, it may seem intuitive that a maintaining a good blood pressure during intensive (ICU) and other areas of critical care is important. Recently, evidence for this is emerging. For example, Maheshwari et al showed that in septic ICU patients risks for mortality, AKI, and myocardial injury were apparent at 85 mmHg, and for mortality and AKI risk progressively worsened at lower thresholds (Maheshwari et al., 2018).

The current main approach to cardiovascular management and maintaining blood pressure is to monitor blood pressure and to treat with volume, vasopressors and inotropes or to adjust anaesthetic levels.

In higher-risk surgical patients and many intensive care patients, blood pressure is usually monitored with a transducer connected to an invasive arterial line. The transducer is in turn connected to a multi-parameter monitor (MPM). This provides a continuous measurement of the arterial blood pressure waveform. The MPM displays to the clinician the waveform, and key current numeric features such as systolic, diastolic and mean arterial pressure, and heart rate.

Although the changes in central venous pressure (CVP) are useful clinical cardiovascular indicators, CVP is not frequently monitored intra-operatively and is falling out of fashion.

In usual peri-operative practice, the anaesthetist closely observes the MPM, along with the anaesthetic machine, ventilation and the current state of the surgical procedure. When the blood pressure falls, the anaesthetist will seek to restore it to higher acceptable levels by treating with volume replacement (eg crystalloids, colloids or blood), vasopressors or inotropes. These treatments may be administered by gravity flow, infusion pumps or manual syringe boluses. One of the most common treatments is to use manual syringe boluses of vasopressor.

Typically, the anaesthetists will respond as the blood pressure falls below certain thresholds such as 65 mmHg or 55 mmHg. The anaesthetist may use different thresholds based on the patient's pre-operative risk. For example, they may be higher if the patient has pre-existing hypertension. It is possible for the anaesthetist to set a patient specific alarm on the MPM when blood pressure falls below a defined value. In practice this is little used, owing to alarm fatigue and the fact that the alarm may be too late after the pressure has fallen.

The anaesthetist will make an assessment of the cause of the falling blood pressure in order to select the appropriate therapy.

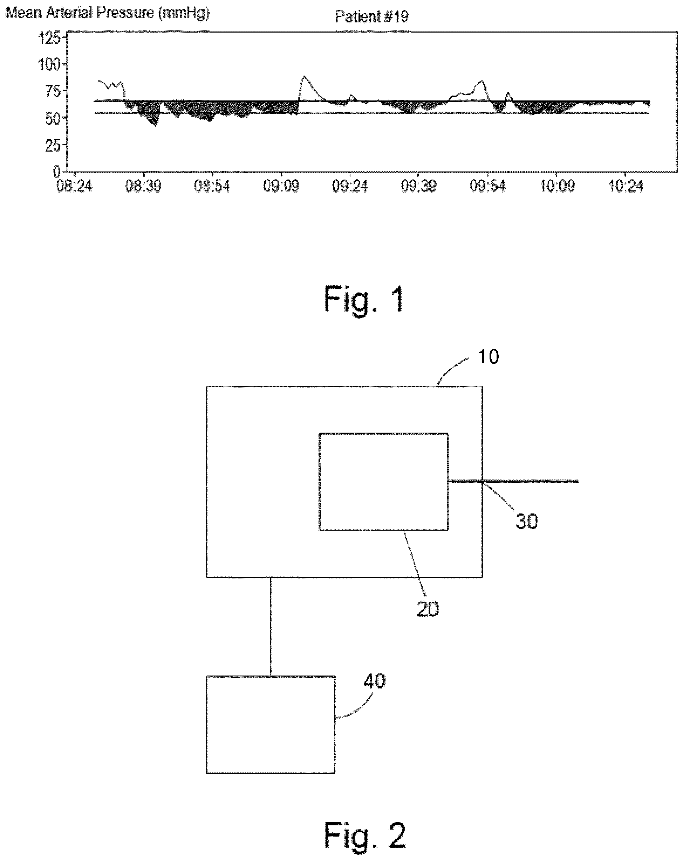

This current practice requires vigilance on the part of the anaesthetist. Whilst this is usually the case, at times the workload is very high and it is not possible to focus on several things simultaneously. This tends to lead to a reactive approach as shown in Figure 1, which shows the first 2 hours of a major gastro-intestinal surgical procedure. The peaks and troughs correspond to the administration of vasopressors or volume in response to the blood pressure falling below certain values. Sometimes this happens at the same time as crossing the threshold, and sometimes minutes after the event.

Similar practices are used in intensive care. However, care is usually given by a clinical team and not a single anaesthetist, with a nurse being the main bedside clinician, and cardiovascular medications infused rather than administered by manual syringe bolus.

The timescales in intensive care are usually longer and changes slower compared to intra-operative care.

Some anaesthetists use cardiac output monitoring during high-risk surgery (estimated at 10-15% of procedures). These techniques estimate blood flow rate from the left ventricle into the systemic circulation.

The composite values and trends of mean arterial blood pressure (MAP) and cardiac output (CO) may be interpreted by the anaesthetist to assess the causes of any cardiovascular changes and to decide appropriate treatment. In principle, this should help reduce unwanted events such as hypotension.

The pulmonary artery catheter (PAC) has been regarded as a gold standard of measurement. However, it is highly invasive and only measured intermittently (eg 30min or 1 hour). The use of the PAC has fallen to very low levels, with cardiac surgery being an exception. Its intermittent nature makes it unsuitable for the hypotension problem where dynamics can change over just a few minutes and very short periods of very low hypotension for even a couple of minutes increase risk of complications.

Other techniques include pulse contour and bio-impedance methods. These methods estimate left heart stroke volume from features of the arterial pressure waveform or electrical impedance changes respectively, and then multiply by heart rate to obtain cardiac output. The pulse contour methods require intermittent calibration in order for the absolute values to have precision.

The introduction of cardiac output monitoring has generally focussed on maintaining or optimising flow (cardiac output), rather than blood pressure. They have been shown to reduce complications (Cecconi, Corredor, et al., 2013).

Importantly, the incidence of intra-operative hypotension and related post-operative complications reported above occurs despite the current availability of MPM and CO technology. This suggests the need for new technology and practice methods to further prevent hypotension.

WO 2011/080190 A1 relates to a monitoring device for prediction of a rapid symptomatic drop in a subject's blood pressure. EP 2 563 213 A relates to a monitoring device arranged to display a level of consciousness and hemodynamic parameters of a patient for simultaneous viewing.

## SUMMARY OF THE INVENTION

The present invention is defined by the independent claims appended hereto. Further advantageous embodiments may be found in the dependent claims, also appended hereto.

We will describe a system for determining relative changes in at least one parameter of cardiovascular function, the system comprising:

an input for receiving arterial blood pressure waveform data; and
signal processing apparatus, coupled to said input and configured to:

detect a series of pulses from the arterial blood pressure waveform data;
calculate at least one baseline cardiovascular parameter value for a first pulse using data from the first pulse and a second pulse within the detected series of pulses, wherein the first pulse is consecutive to the second pulse;
calculate at least one cardiovascular parameter value for a plurality of pulses within a time period comprising a sequence of n pulses in the arterial blood pressure waveform data;
determine a time averaged mean of each of the plurality of cardiovascular parameter values
for each of the time averaged mean cardiovascular parameter values, calculate the relative change of the respective time averaged mean cardiovascular parameter value based on the difference between the respective time averaged mean cardiovascular parameter value and the baseline cardiovascular parameter.

This value of relative change over time for each of the points can then be used to show a likely trend, for example in the blood pressure, that would indicate that, if no action were taken, the patient may experience hypotension in the immediate future.

The input for receiving arterial blood pressure waveform data may comprise an indwelling catheter such an arterial catheter or a non-invasive method of monitoring arterial pressure. These provide a less invasive method of receiving arterial blood pressure waveform data and simplify measurement of the blood pressure waveform data.

There may be a predetermined and constant time period between the first pulse of the detected series of pulses and the sequence of n pulses. This means that the baseline cardiovascular parameter value may not always be calculated at an initial time period (for example, where T=0), but may be dynamic and measured a set time period prior to the sequence of n pulses used for calculating the time averaged cardiovascular parameter value. This allows greater ability to identify, view, and react to recent trends in cardiovascular function rather than always comparing to a static baseline value.

The time averaged mean of the cardiovascular parameter values may also be calculated for a plurality of sequences of pulses between a present time and the first and second pulses used to calculate the baseline parameter value. The device may calculate the relative change for each of the plurality of sequences, such that a series of relative change values are calculated compared to the baseline value. This allows a user to view and identify changes since the first and second pulses used to calculate the baseline parameter value.

The time period between the between the first pulse of the detected series of pulses and the sequence of n pulses may be the same as the time period comprising a the sequence of n pulses. In other words, the time averaged mean cardiovascular parameter values over a given time period are compared with the baseline cardiovascular parameter value at the start of the latest time period, where the latest time period may have the same length as the given time period. For example, both the given time period and the latest time period may be a five minute time period.

Alternatively, the time period between the between the first pulse of the detected series of pulses and the sequence of n pulses may be different to the time period comprising a the sequence of n pulses. The time averaged mean cardiovascular parameter values over a given time period are compared with the baseline cardiovascular parameter value at the start of the latest time period, where the latest time period may be longer than the given time period.

The at least one parameter of cardiovascular function may comprise nominal cardiac output. This relative change in nominal cardiac output is approximately the same relative change that would be calculated if a calibrated cardiac output value had been used, as the calibration coefficient approximately cancels out in the percentage difference formula between baseline and current values.

The at least one parameter of cardiovascular function may comprise systemic vascular resistance. The signal processing apparatus may be further configured to calculate the relative change in systemic vascular resistance using the relative change in nominal cardiac output.

The relative change in systemic vascular resistance may be calculated by dividing a determined time average mean of mean arterial blood pressure by the relative change in nominal cardiac output.

The relative change in systemic vascular resistance may be calculated by:

calculating a baseline total systemic vascular resistance for a first pulse;
calculating total systemic vascular resistance for a plurality of pulses within a time period comprising a sequence of n pulses in the arterial blood pressure waveform data:

determining a time averaged mean of total systemic vascular resistance;
for each of the time averaged mean total systemic vascular resistance values, calculating the relative change of the respective time averaged mean total systemic vascular resistance based on the difference between the respective time averaged mean total systemic vascular resistance value and the baseline total systemic vascular resistance value.

Total systemic vascular resistance may be calculated by dividing mean arterial blood pressure by nominal cardiac output.

The at least one parameter of cardiovascular function may comprise venous return driving pressure. The signal processing apparatus may be further configured to calculate the relative change in venous return driving pressure using nominal cardiac output and mean arterial blood pressure. Venous return driving pressure provides further information to a user, beyond mean arterial blood pressure, cardiac output, and systemic vascular resistance.

The relative change in venous return driving pressure may be calculated from the relative change in cardiac output and a change in mean arterial pressure.

The system may further comprise a memory configured to store input arterial blood pressure waveform data and parameter of cardiovascular function data.

The signal processing apparatus may be further configured to determine if the at least one cardiovascular parameter is within a predetermined range.

The system may be configured to alert a user if said at least one cardiovascular parameter is outside the predetermined range.

The signal processing apparatus may be further configured to assign a signal abnormality index value to a pulse in the sequence of n pulses dependent upon the outcome of said determination. The pulse may correspond to a pulse for which the at least one cardiovascular parameter is outside the predetermined range.

The signal processing apparatus may be further configured to determine a length of time the at least one cardiovascular parameter is outside the predetermined range.

The signal processing apparatus may be further configured to extrapolate the at least one cardiovascular parameter to a future time.

The signal processing apparatus may be configured to determine if a statistical trend is present in the at least one cardiovascular parameter. The signal processing apparatus may be configured to output the extrapolated cardiovascular parameter at a future time dependent upon said outcome of determination of statistical trend.

The signal processing apparatus may be configured to extrapolate the at least one cardiovascular parameter at a future time using a linear regression technique.

The signal processing apparatus may be configured to determine if a statistical trend is present in the at least one cardiovascular parameter by calculating the number of standard deviations of the cardiovascular blood pressure parameter about a regression line and determining if the number of standard deviations is greater than a predetermined threshold.

The signal processing apparatus may be configured to extrapolate the at least one cardiovascular parameter at a future time if a treatment is administered.

The system may be configured to alert a user if said extrapolated at least one cardiovascular parameter at a future time is outside the predetermined range.

The system may further comprise a user interface configured to display the relative change in at least one parameter of cardiovascular function.

The signal processing apparatus may be configured to remove artefacts from the arterial blood pressure waveform data. These removes, for example, artefacts due to line flushing or transducer zeroing.

The system may be configured to selectively output the calculated relative change of the time averaged mean cardiovascular parameter value dependent upon the signal quality of the arterial blood pressure waveform data received from the input. The system may output the calculated relative change of the time averaged mean cardiovascular parameter value only when the signal quality of the arterial blood pressure waveform data is above a threshold value.

We will also describe a method of determining relative changes in at least one parameter of cardiovascular function, the method comprising:

receiving arterial blood pressure waveform data;

detecting a series of pulses from the arterial blood pressure waveform data;

calculating at least one baseline cardiovascular parameter value for a first pulse using data from the first pulse and a second pulse within the detected series of pulses, wherein the first pulse is consecutive to the second pulse;

calculating at least one cardiovascular parameter value for a plurality of pulses within a time period comprising a sequence of n pulses in the arterial blood pressure waveform data;

determining a time averaged mean of each of the plurality of cardiovascular parameter values; and

for each of the time averaged mean cardiovascular parameter values, calculating the relative change of the respective time averaged mean cardiovascular parameter value based on the difference between the respective time averaged mean cardiovascular parameter value and the baseline cardiovascular parameter value.

We will also describe a method of preventing or treating hypotension, comprising:

determining the relative changes in at least one parameter of cardiovascular function according a method as described above; and

administering a treatment in response to said relative changes in at least one parameter of cardiovascular function.

LIST OF FIGURES

The present invention will now be described, by way of example only, and with reference to the accompanying figures, in which:

Figure 1 shows a plot of the Mean Arterial Pressure over a 2 hour time period during a major gastro-intestinal surgical procedure;

Figure 2 shows a simplified systems diagram;

Figure 3 shows an example implementation of the software architecture;

Figure 4 shows an example beat detection, feature extraction and signal quality checking algorithm;

Figure 5 shows the beat detection algorithm in more detail; and

Figure 6 shows an example User Interface for the system.

DETAILED DESCRIPTION OF THE PREFERRED EMBODIMENTS

The technology and innovations described here are intended to enable the creation of clinical decision support systems and semi- and fully-automated care systems for reducing the incidence of low blood pressure (hypotension) and improving cardiovascular stability during surgical procedures and in other critical care environments such as intensive care, emergency departments and casualty care.

In brief, the present invention provides a system for determining relative changes in at least one parameter of cardiovascular function. Ultimately, the present invention provides an output showing a relative change of the parameters associated with cardiovascular function over a period of time. This relative change is between a time averaged mean of cardiovascular parameters (for each pulse within a series of pulses from an arterial blood pressure waveform data) and a baseline cardiovascular parameter value based on first and second pulses within the blood pressure waveform data.

Figure 2 shows a very simplified functional diagram of the system.

In its most fundamental form, the system 10 comprises an input 30 for receiving arterial blood pressure waveform data, a signal processor 20 that may receive the arterial blood pressure waveform data and that is configured to process the data. A display 40 may be coupled to this system in order to display the resulting output that assists in the determination of which action to take.

The input 30 may be a patient monitor; which can include a catheter, such as an indwelling catheter or arterial line connected to a pressure transducer.

The signal processor may perform a number for functions or calculations. The signal processor may be a single unit performing a plurality of functions or calculations, or a plurality of units communicating with each other to perform the plurality of functions or calculations.

The arterial blood pressure waveform data comprises a sequence of data for a patient's blood pressure over a period of time. Preferably the waveform data is a continuous stream of blood pressure waveform data, or it may be a block of data.

The signal processor is configured to detect a series of pulses from the arterial blood pressure waveform data (this will be described in more detail below).

The signal processor then calculates a baseline cardiovascular parameter value for a first pulse using data from the first pulse and a second pulse within the detected series of pulses. The first pulse and second pulses are consecutive pulses. The baseline data provides a baseline value against which other values are compared. The cardiovascular parameter value is nominal cardiac output. Systemic vascular resistance may also be calculated.

The signal processor calculates at least one cardiovascular parameter value for a plurality of pulses within a time period comprising a sequence of n pulses in the arterial blood pressure waveform data. This cardiovascular parameter values for the plurality of pulses is then used to determine a time averaged mean of each of the plurality of cardiovascular parameter values.

The signal processor then calculates, for each of the time averaged mean cardiovascular parameter values, a relative change of the respective time averaged mean cardiovascular parameter value based on the difference between the respective time averaged mean cardiovascular parameter value and the baseline cardiovascular parameter value.

This value of relative change over time for each of the points can then be used to determine what action to take and when to take it, since the relative change over time will show a likely trend for example in the blood pressure that would indicate that, if no action were taken, the patient may experience hypotension in the immediate future. When the cardiovascular parameter value is systemic vascular resistance, the signal processing apparatus is configured to calculate the relative change in systemic vascular resistance using the relative change in nominal cardiac output.

When the cardiovascular parameter value is venous return driving pressure, the signal processing apparatus is further configured to calculate the relative change in venous return driving pressure using nominal cardiac output and mean arterial blood pressure. Based on these trends from the relative change over time compared to a baseline, methods of treatment for the possible onset of hypotension may be administered in good time to prevent those conditions from occurring. This may be done through notification to a suitably qualified clinician operating the system. Automatic or semiautomatic systems for the administering of medicaments in response to the output of the system is possible.

The technology is intended to provide an assistive decision support system that helps clinicians prevent hypotension by reacting earlier with the right treatment.

Some of the advantages of this system are presented below:

- Better situation awareness
- A focus on arterial blood pressure, especially mean arterial pressure (MAP)
- Setting of patient-specific targets
- Projection of how MAP is likely to evolve over the next period, in relation to the patient specific targets
- A panel of recent changes in associated cardiovascular parameters that informs the clinician about the underlying causes and thus appropriate treatment
- Labelling of physiological data with treatment interventions to enable the clinician to better assess the patient response to different treatments
- Computation of cumulative time patient is in different MAP ranges, associated with different levels of post-operative complication risk

Below is a summary of components and functions that may be advantageous in the formation of the system:

- A medical touch-screen computer connected to the multi-parameter monitor (MPM)
- Software performing the following functions:

  ○ Communication with the MPM to acquire arterial blood pressure waveform data continuously
  ○ Beat detection, feature extraction and signal quality checking
  ○ Censoring beats and periods of signal where there are signs of signal abnormalities
  ○ Remove noise by filtering the signal after abnormality censoring
  ○ Compute derived parameters based on the filtered values
  ○ A user interface to allow user input and to display numeric and graphical values

- Use of the Liljestrand and Zander algorithm (LZA, (Liljestrand and Zander, 1928)) to estimate cardiac output (CO) and in turn use this to estimate systemic vascular resistance (SVR)

◦ Restrict the display of CO and SVR information to relative changes over a recent period of time (eg 5 minutes) rather than using absolute values

◦ The advantages of this approach are that LZA has been shown to be the most reliable of pulse contour CO algorithms, it is most precise for changes in CO than absolute values (Broch et al., 2016a) and that CO and SVR relative changes are more meaningful to clinicians in assessing the patient and deciding treatment than absolute values

• Compute an estimate of the driving pressure for venous return (Pvr) and its changes over time. This provides the anaesthetist with an assessment of the changes in the volume state and cardiac function of the patient

• Statistical methods to detect MAP trends over the recent past (usually 2 minutes). If there are consistent trends to project them forward, compared to targets, for the next period (usually 2 minutes)

• Computation of cumulative times in different MAP ranges that correspond to different risks of hypotensive post-operative complications

• An innovative user interface design method:

◦ Gives prominence to MAP as the primary parameter
◦ Highlights MAP compared to a clinician-set target range
◦ Includes multiple simultaneous timescales to aid the clinician in assessing the patient and deciding treatment:

■ 20 minutes default MAP history adjustable to the whole procedure duration
■ 5 minutes of recent trends in the casual cardiovascular parameters including CO, SVR and heart rate (HR)
■ 2 minutes forward projection of MAP trend compared to targets
◦ Display of cumulative time in MAP risk ranges
◦ Ability to add treatment markers to the physiological trends in order to show the effects of different treatments

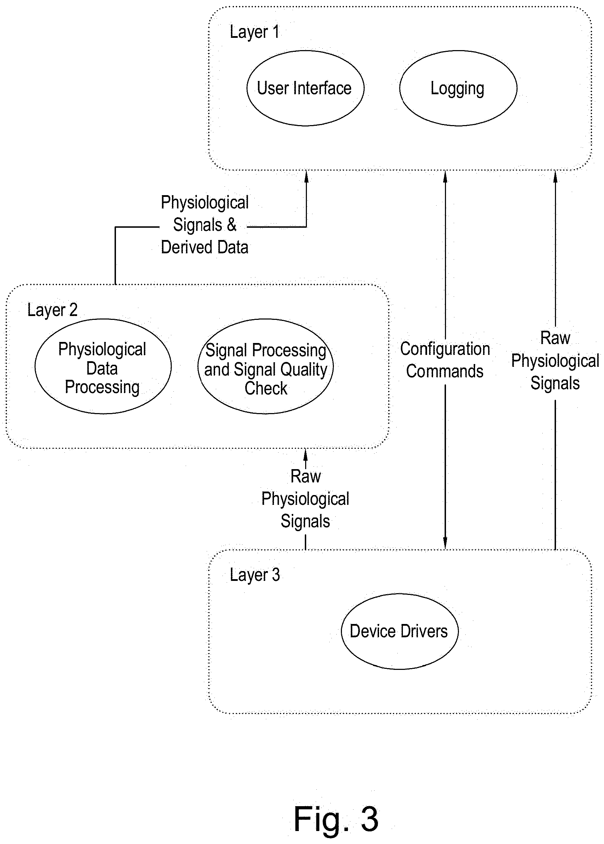

Figure 3 shows an example implementation of the software architecture. It preferably consists of the following items:

• User Interface: Display patient data and derived data trends and forecasts. Frontend displays data and receives user input but does not perform any physiological calculations.
• Device Drivers: Implement communications with multiparameter monitors. This item provides a stream of real-time data in a common data format.
• Signal Processing and Signal Quality Checking: Perform any processing on data from the device drivers and signal quality assurance.
• Physiological Data Processing: Computation of derived physiological parameters from quality-checked physiological signals.
• Patient Data and User Input Logging: User interactions, all displayed data in the User Interface, and raw input signals to be logged to the disk.

We will now go into more detail of the processing of the arterial blood pressure waveform data in order to provide the required trending data upon which a determination of the patient's condition may be made.

Beat Detection, Feature Extraction and Signal Quality Checking

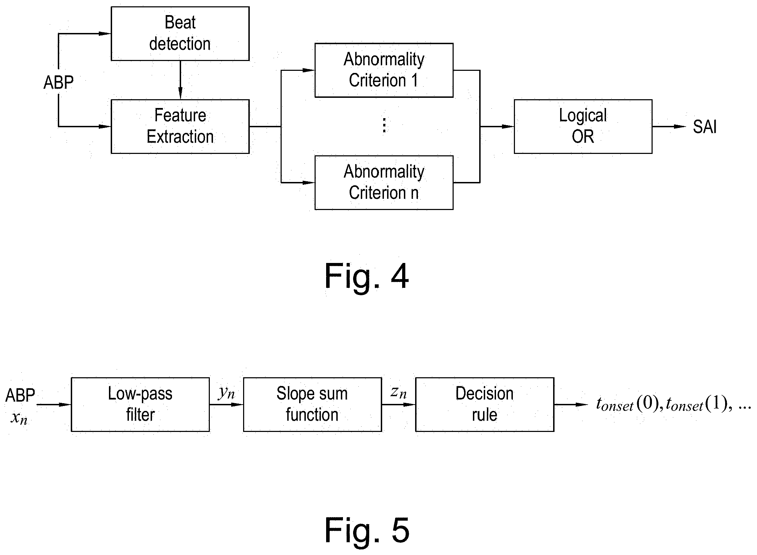

The beat detection, feature extraction and signal quality checking algorithm follows that of Sun et al (Sun, Reisner and Mark, 2006) and consists of several components as shown in Figure 4. (This algorithm has been shown to have a sensitivity of 1 and specificity of 0.91 compared to an expert annotator.)

ABP is the sampled arterial blood pressure waveform, and SAI is the signal abnormality index. The ABP is a signal sampled at a rate between 100 Hz and 250 Hz, and obtained from the MPM. The beat detection algorithm follows that of (Zong *et al.,* 2003). (In comparison with 39,848 beats in a reference database, the difference between manually edit and algorithm determined ABP pulse onset was less than or equal to 20 ms).

The beat detection algorithm consists of three components: a low-pass filter, a windowed and weighted slope sum function, and a decision rule.

Figure 5 shows the beat detection algorithm in more detail.

$x_n$ is the input of the low-pass filter and $y_n$ is the filtered ABP. The slope sum function converts $y_n$ to a slope sum signal $z_n$. A decision rule is applied to $z_n$ to determine the ABP pulse onsets denoted by $t_{onset}(0), t_{onsee}(1), \ldots, t_{onset}(k), \ldots$

**Low-pass filter.** The purpose of the low-pass filter is to suppress high frequency noise that might affect the ABP onset detection. The following second order recursive filter may be used:

$$y_n = 2y_{n-1} - y_{n-2} + (x_n - 2x_{n-5} + x_{n-10})/25$$

At a sampling rate of 250 Hz, this has a 3dB cut-off of about 16 Hz, at 125 Hz the 3 dB cut-off is about 8 Hz and at 100 Hz the 3dB cut-off of about 7 Hz. The gain is 1x at 0 Hz. The phase shift is 20 ms at a sampling rate of 250Hz (equivalent to 5 samples). The phase shift is 32 ms at a sampling rate of 125Hz (equivalent to 4 samples). The phase shift is 40 ms at a sampling rate of 100 Hz. A phase adjustment should be made of 4 samples at 125Hz and 4 samples at 100Hz.

The time precision of heart period estimates from discrete time sampled data is 4 ms for 250 Hz, 8 ms for 125 Hz and 10 ms for 100 Hz sampling respectively.

**Slope-sum function.** The purpose of the slope-sum function (SSF) is to enhance the upslope of the ABP pulse and to suppress the remainder of the waveform. The windowed and weighted SSF at time $i$, $z_i$ is defined as:

$$z_i = \sum_{k=i-w}^{i} \Delta u_k, \quad where \ \Delta u_k = \begin{cases} \Delta y_k : \Delta y_k > 0 \\ 0 : \Delta y_k \leq 0 \end{cases}$$

Where w is the length of the analyzing window. Zong et al (2003) use w = 128 ms or 32 samples for a sampling frequency 250 Hz. For a sampling frequency of 125 Hz, 16 samples is used. For a sampling frequency of 100 Hz, 13 samples is used.

The onset of the SSF pulse generally coincides with the onset of the ABP pulse as the SSF signal can only rise when the ABP signal rises.

**Decision rule.** The SSF is compared with a *threshold* to identify a potential ABP pulse onset. The *threshold* is defined as 60% of a threshold base value. The initial value of the threshold base value is three times the mean SSF signal averaged over the first 10 seconds of signal.

When the SSF crosses the *threshold* at a crossing point:

- Search back -150 ms for the minimum value of SSF, $z_{min}(k)$ and search forward +150 ms for the maximum value of SSF, $z_{max}(k)$ at $t_{max}(k)$;
- If $z_{max}(k)$ - $z_{min}(k)$ > *threshold* then accept the pulse detection for pulse $k$, else reject the pulse detection;
- If pulse detection is accepted, then:

    ○ Search backwards to the point where SSF exceeds 2.5% of the maximum value - this is the onset point, $t_{onset}(k)$;
    ○ Search forward +150 ms for the maximum value of raw ABP, $x(k)$ at systolic $t_{sys}(k)$;
    ○ Adjust calculated ABP onset time, $t_{onsee}(k)$, by the phase lag (20 ms or 5 samples at 250 Hz sampling rate, 32 ms or 4 samples at 125 Hz sampling rate, 40 ms or 4 samples at 100 Hz sampling rate) to give diastolic $t_{dia}(k)$ and systolic $t_{sys}(k)$ times;
    ○ **Feature Extraction.** Compute the features of the previous pulse $k$ - 1 for which the onset time marks the close:

        ▪ Look up from the raw ABP wave $x_n$, diastolic pressure $P_{dia}(k$ - $1)$ at diastolic time $t_{dia}(k$ - $1)$ and systolic pressure $P_{sys}(k$ - $1)$ at systolic time $t_{sys}(k$ - $1)$;
        ▪ Compute heart period for this beat as

$$T_H(k - 1) = t_{dia}(k) - t_{dia}(k - 1) \text{ sec};$$

        ▪ Compute pulse rate as

$$HR(k - 1) = f(k - 1) = 60/T_H(k - 1) \text{ bpm};$$

        ▪ Compute pulse pressure

$$P_{pulse}(k - 1) = P_{sys}(k - 1) - P_{dia}(k - 1);$$

        ▪ Compute the noise of the pulse $k$ - 1, $\eta(k$ - $1)$, as the average of the negative slopes over the full pulse, using units of mmHg/100ms.

○ Apply a 300 ms "eye-closing" window to avoid double crossing. The eye closing window will start from the pulse onset time;

**Abnormality indexing.** With blood pressure features available, they are compared with the following table. If any one criteria is met, the signal abnormality index SAI is set to 1 for this beat.

| Feature | Abnormality Criterion | Type |
|---|---|---|
| $P_{sys}$ | $P_{sys} > 300$ *mmHg* | Physiological range |
| $P_{dia}$ | $P_{dia} < 20$ *mmHg* | Physiological range |
| $P_{pulse}$ | $P_{pulse} < 20$ *mmHg* | Physiological range |
| MAP | MAP < 30 *or* MAP > 200 *mmHg* | Physiological range |
| f | f < 20 or f > 200 *bpm* | Physiological range |
| $\eta$ | $\eta < -40$ *mmHg*/100ms | High frequency noise present |
| $\Delta P_{sys} = P_{sys}[k] - P_{sys}[k-1]$ | $|\Delta P_{sys}| > 20$ *mmHg* | Maximum change between 2 pulses |
| $\Delta P_{dia} = P_{dia}[k] - P_{dia}[k-1]$ | $|\Delta P_{dia}| > 20$ *mmHg* | Maximum change between 2 pulses |
| $\Delta T = T_H[k] - T_H[k-1]$ | $|\Delta T| > 2/3$ *sec* | Maximum change between 2 pulses |

**Update Threshold.** If the beat is not abnormal, then update the threshold to current SSF maximum value $threshold(k+1) = 0.9 * threshold(k) + 0.1 * 0.6 * z_{max}(k)$.

Filtering

Physiological signals to be used in further processing or for display are filtered with a time-moving mean filter with a time window of $T_{filt}$ seconds (30 sec). A bar ( ) is used as an accent over a variable X to denote the mean value. The subscript *Now* denotes the most current time. The mean filter is

$$\overline{X_{Now}} = mean\left((X(t_k) \mid t_k \in [t_{Now} - T_{filt}, t_{Now}] \wedge X(t_k) \neg\, abnormal\right)$$

(In words, "the mean $\overline{X_{Now}}$ is the mean of all $X(t_k)$ where $t_k$ is in the range from $T_{filt}$ seconds ago until the current time $t_{Now}$ excluding all $X(t_k)$ which have been labelled abnormal in the signal quality checking".)
If there are less than $N_{MedMin}$ non-abnormal values in the filter, the filter returns NaN ("not a number"). This indicates that there is insufficient quality data to estimate a filtered value. $N_{MedMin}$ will take the value of 6.
The mean filter is applied to mean arterial pressure, systolic pressure, diastolic pressure and heart rate as follows:

$$\overline{MAP} = mean(MAP)$$
$$\overline{P_{sys}} = mean(P_{sys})$$
$$\overline{P_{dia}} = mean(P_{dia})$$
$$\overline{HR} = mean(f)$$
$$\overline{T_H} = mean(T_H)$$
$$\overline{P_{pulse}} = mean(P_{pulse})$$

For simplicity the subscript *Now* has been omitted in the above, and it is understood that the current $t_{Now}$ value is used in subsequent processing.
Pulse pressure variation (*PPV*) is computed over the last $T_{PPV}$ seconds (30 seconds), as follows:

$$PP_{Max} = Max\left((P_{Pulse}(t_k) \mid t_k \in [t_{Now} - T_{PPV}, t_{Now}] \wedge P_{Pulse}(t_k) \neg\, abnormal\right)$$

(In words, "the maximum pulse pressure $PP_{Max}$ is the maximum of all pulse pressures $P_{Pulse}(t_k)$ where $t_k$ is in the range from $T_{PPV}$, seconds ago until the current time $t_{Now}$ excluding all $P_{Pulse}(t_k)$ which have been labelled abnormal in the signal quality checking".)

$$PP_{Min} = Min\left( (P_{Pulse}(t_k) \mid t_k \in [t_{Now} - T_{PPV}, t_{Now}] \wedge P_{Pulse}(t_k) \neg\, abnormal \right)$$

$$PPV_{Now} = \frac{PP_{Max} - PP_{Min}}{(PP_{Max} + PP_{Min})/2}$$

$T_{PPV}$ usually takes the value 30 seconds. If there are less than $N_{PPV_{Min}}$ non-abnormal values in the *Max* and *Min* functions, $PPV_{Now}$ is set to NaN ("not a number"). This indicates that there is insufficient quality data to estimate a filtered value. $N_{PPV_{Min}}$ will take the value of 6.

Derived Variables

Derived variables are computed using the current values of the mean filtered values as specified above.

Nominal Cardiac Output (nCO)

Nominal cardiac output (*nCO*) is estimated using the Liljestrand and Zander (LZA) method (Liljestrand and Zander, 1928; Broch *et al.,* 2016b).

$$nCO = \frac{k_{LZ}\overline{HR}(\overline{P_{sys}} - \overline{P_{dia}})}{(\overline{P_{sys}} + \overline{P_{dia}})}$$

$k_{LZ}$ is a calibration coefficient for the LZ method. A value of 0.42 is used. This is based on a blood pressure of 120/80 mmHg with a heart rate of 60 bpm and a cardiac output of 5 L/min.
It is important to note that only relative % changes in nominal cardiac output (*nCO*) are displayed and not absolute values.
If one or more of the input values are NaN, *nCO* is set to NaN.
The values of nCO over a given time period (for example, five minutes) are compared with a baseline value at the start of the latest time period (five minute period) (ΔCO) and expressed as a percentage change which is plotted on the display. The calculated baseline value is therefore dynamic and changing as the latest time period changes. In this example, the value of the latest time period is a 5 minute interval, and the percentage change is shown as a percentage relative to the baseline across the whole 5 minutes. In this manner, ΔCO can be not only shown as a single number change, but a series of ΔCO across the latest 5 minutes time period. This allows a user to view and identify changes within this latest 5 minutes time period.
The latest time period may be 5 minutes, however it could be less or more - for example, the latest time period could be up to 1 hour.
The percentage difference between the current and baseline values is also displayed numerically. This percentage difference is approximately the same percentage difference that would be calculated if a calibrated CO value had been used, as the calibration coefficient approximately cancels out in the percentage difference formula between baseline and current values. If there are periods where the arterial pressure signal quality is too low to compute ΔCO, the ΔCO trend chart will include gaps.
The accuracy of the Liljestrand and Zander formula in estimating cardiac output and its trend changes has been evaluated by Sun et al., (2009) , Monge Garcia et al., (2013) , Zhang et al., (2015) and Caillard et al., (2015).

Systemic Vascular Resistance (*SVR*)

The system uses a measure of total systemic vascular resistance (TSVR) to compute the recent changes in systemic vascular resistance (SVR). TSVR is defined as:

$$TSVR = 80 * \frac{MAP}{CO}$$

The conventional definition is:

$$SVR = 80 * \frac{MAP - CVP}{CO}$$

Atlas *et al.,* (2010) showed mathematically that changes in TSVR are approximately equal to changes in SVR ($\Delta$TSVR $\approx \Delta$SVR).

The systemic vascular resistance (SVR) is computed using nominal cardiac output (*nCO*) as follows:

$$SVR = \frac{\overline{MAP}}{nCO}$$

BP Assist will only use changes in systemic vascular resistance (SVR) and not absolute values.

The values of SVR over the last five minutes are compared with a baseline value at the start of the latest five minute period ($\Delta$SVR) and expressed as a percentage change which is plotted on the display.

If one or more of the input values are NaN, *SVR* is set to NaN.

Venous Return Driving Pressure (Pvr)

The primary purpose of this technology is to highlight key trends in blood pressure, compared to target or threshold levels, and to project these forward. If blood pressure is about to drop too low, this is a call to action to the clinician to intervene. Low blood pressure itself may be an indication for giving a pressor bolus or increasing the pressor infusion rate. Similar arguments apply if the blood pressure is trending too high.

In deciding treatment, the clinician should consider all the key aspects related to blood pressure including the induction and depth of anaesthesia, the stage of surgery, blood loss, etc.

From a haemodynamic point of view, the causes of a falling blood pressure are reduced vasomotor tone (vasodilation, eg caused by anaesthesia), reduced volume state (eg through bleeding) or reduced heart function.

Most clinicians can make this assessment and decide treatment by analysing the patterns of changes in blood pressure (MAP), cardiac output (CO) and systemic vascular resistance (SVR). However changes in each of these variables can be caused by changes in any of the underlying states: volume, tone or heart. It would be helpful, therefore, to provide further information to help clinicians make this decision.

Guyton coined the term "mean systemic filling pressure" ($P_{ms}$) for the pressure of an average element in the circulation that drives blood back to the heart. This is not simply the average of arterial and venous pressures. Actually, it is the pressure that the circulation would equilibrate to if the heart was stopped. $P_{ms}$ is a result of the contained volume of blood stressing an elastic set of tubes in the circulation, and hence a measure of "how well filled the circulation is" - or volume state.

A measure of $P_{ms}$ would be very useful clinically. Clearly, stopping the heart is not a desirable nor practical method. Parkin (Parkin *et al.,* 1994) had the insight that a simple mathematical model could be fitted to the current patient parameters, and the heart stopped in the model, rather than the patient. His analysis yielded the following formula for estimating $P_{ms}$,

$$P_{msa} = aRAP + bMAP + cCO$$

Where $P_{msa}$ is referred to as the "mean systemic filling pressure analogue". *RAP* is right atrial pressure, *MAP* is mean arterial pressure and *CO* is cardiac output. a and b are fixed constants, with values a=0.96 and b=0.04. The coefficient c is a function of age, height and weight, ranging from 0.6 (young and large adult) to 1.3 (old, frail, small adult). Note that central venous pressure (CVP) may be used to replace RAP in this formula.

The Parkin $P_{msa}$ formula has been independently validated (Maas *et al.,* 2012; Cecconi, Aya, *et al.,* 2013; Lee *et al.,* 2013) and was used as the basis of the Navigator clinical decision support system (Parkin and Leaning, 2008; Pellegrino *et al.,* 2011; Sondergaard *et al.,* 2012).

As noted earlier, CVP (and RAP) are not routinely monitored in surgical procedures. Further, it is becoming a less popular measurement in intensive care, where it requires careful quality control, including levelling of the transducer when the patient moves. This makes the Parkin $P_{msa}$ formula limited in its application.

Nonetheless the Parkin formula can be used to shed light on whether there are changes in volume state or heart function.

The difference between mean systemic pressure $P_{ms}$ and right atrial pressure RAP is the driving pressure for venous return, $P_{vr}$:

$$P_{vr} = P_{ms} - RAP$$

Note that if the volume state decreases, it follows from this formula that $P_{vr}$ will also drop. Hence decreases in $P_{vr}$ may be an indicator for reduced volume state.

When heart function decreases for a given volume state, it also follows that $P_{vr}$ will drop as $RAP$ will rise. Hence decreases in $P_{vr}$ may be an indicator for decreased heart function.

The change $\Delta P_{vr}$ may thus be a useful clinical parameter in addition to MAP, CO, SVR and HR.

We note that in the $P_{msa}$ formula, the coefficient a has the value 0.96, hence the RAP term approximately cancels so that:

$$\Delta P_{vr} = b\Delta MAP + c\Delta CO$$

This parameter does not depend on the absolute value of CO, only its change.

## Recent Changes

Recent changes in $nCO$, $\widetilde{HR}$, $SV$ and $P_{vr}$ nay be computed over a period of $T_{delta}$ before the current time $t_{Now}$.

Their values at $t_{Now}$ - $T_{delta}$ is used as baseline values.

Intermediate values between $t_{Now}$ - $T_{delta}$ and $t_{Now}$ is computed and expressed as a percentage of their baseline values. There is a sufficient number of intermediate points as required graphically.

$T_{delta}$ is typically 300 seconds (5 minutes).

## Cumulative Time Under Thresholds

Total time in minutes under the different thresholds in the patient mean arterial pressure (*MAP*) target range and the warning threshold is computed from the start of the receipt of data from the patient monitor.

## MAP Projection

The method computes and project (extrapolate) a trend and uncertainty band based on the last few minutes of mean arterial blood pressure.

The concept is that if there is a confirmed statistical trend in the signal it will be projected. If there is no confirmed statistical trend, the projection will not be made.

Any statistical trend method that is able to yield a measure of effective fit and uncertainty bands of projection may be used, as long as the method is adequately determined by the data.

Below we use a linear regression technique to evaluate whether the filtered MAP signal is following a consistent linear trend. We have found this to provide a high accuracy projection technique (>90%) on real surgical datasets, as shown below.

The performance during periods of increasing or decreasing MAP was evaluated to cover the 60-110 mmHg range of MAP.

The performance of the projection feature was evaluated by:

1. Bench testing by creating such situations using a calibrated source of analog arterial blood pressure waveform signals. The Rigel UNI-SIM was programmed with a sequence of systolic (SBP) and diastolic (DBP) blood pressure changes to shape the output waveform and change MAP. The sequences corresponded to 3 situations during which the projection line should be visible (a relatively stable MAP, MAP increasing rapidly (> 4 mmHg/min), MAP decreasing rapidly (< -4 mmHg/min)) and 2 situations in which the projection line is not expected to be displayed continuously (highly variable MAP and MAP undergoing a direction change).

2. Re-analyzing the processed log file data sets from the system verification. The accuracy of the projection was analyzed in terms of how fast the MAP is changing, confirmation that the projection was not displayed during periods of high MAP variance or direction change and evaluation of the uncertainty cone that appears with the projection line.

## Bench Testing

Tests were done using a Rigel UNI-SIM (30L-0268) vital signs simulator that provides an electrical simulation of a pressure transducer and is used as a reference signal, a Philips Healthcare MX 550 monitor, GE Healthcare CARESCAPE Monitor 450, and Serial cables to connect to the vital signs monitor's digital data export outputs.

The test data for the bench testing verification of the projection function was the calibrated source of analog arterial blood pressure waveform signals produced by the Rigel UNi-SIM (30L-0268) vital signs simulator. The test data for the re-analysis of the system verification were the log files that were produced when the data sets were processed in the original system verification. Three datasets were used.

The vital signs simulator was configured to produce arterial pressure traces that vary over time as specified in 5 scenarios of:

1. MAP relatively stable
2. MAP very fast increase (> 4 mmHg/min)
3. MAP very fast decrease (< -4 mmHg/min)
4. MAP fast noisy increase (projection should not display)
5. MAP turning point (projection should not display during the turning point)

The determined projection values were compared to values that are independently calculated, and are shown below in Tables 1 and 2.

Table 1: The results for this test (scenarios 1-3) for the GE Monitor

| Time from Start (Secs) | Visual Comparison | | HDA Log File Test | | | |
|---|---|---|---|---|---|---|
| | Projection On (Yes /No) | Trend Aligns (Yes / No) | Projected Value (mmHg) | Calculated Values (mmHg) | Difference (mmHg) | Acceptance Criteria (+/-4mmHg) |
| Scenario 1 Relatively Stable | | | | | | |
| 90 | Yes | Yes | 101.7 | 100.5 | 1.2 | Pass |
| 120 | Yes | Yes | 101.6 | 101.5 | 0.1 | Pass |
| 150 | Yes | Yes | 100.2 | 100.2 | 0 | Pass |
| 180 | Yes | Yes | 101.5 | 101.5 | 0 | Pass |
| Scenario 2 Fast Increase (> 4mmHg/min) | | | | | | |
| 90 | Yes | Yes | 87.1 | 87.2 | 0.1 | Pass |
| 120 | Yes | Yes | 90.6 | 90.7 | 0.1 | Pass |
| 150 | Yes | Yes | 93.7 | 93.8 | 0.1 | Pass |
| 180 | Yes | Yes | 98.1 | 98.3 | 0.2 | Pass |
| Scenario 3 Fast Decrease (> 4mmHg/min) | | | | | | |
| 90 | Yes | Yes | 72.8 | 72.1 | -0.7 | Pass |
| 120 | Yes | Yes | 70.6 | 70.5 | -0.1 | Pass |
| 150 | Yes | Yes | 66.9 | 36.8 | -0.1 | Pass |
| 180 | Yes | Yes | 68.6 | 38.6 | 0 | Pass |

Table 2: The results for this test (scenarios 1-3) for the Philips Monitor

| Time from Start (Secs) | Visual Comparison | | HDA Log File Test | | | |
|---|---|---|---|---|---|---|
| | Projection On (Yes /No) | Trend Aligns (Yes / No) | Projected Value (mmHg) | Calculated Values (mmHg) | Difference (mmHg) | Acceptance Criteria (+/-4mmHg) |
| Scenario 1 Relatively Stable | | | | | | |
| 90 | Yes | Yes | 106.2 | 106.2 | 0 | Pass |
| 120 | Yes | Yes | 101.1 | 101.2 | 0.1 | Pass |
| 150 | Yes | Yes | 102.9 | 102.9 | 0 | Pass |

(continued)

| Time from Start (Secs) | Visual Comparison | | HDA Log File Test | | | |
|---|---|---|---|---|---|---|
| | Projection On (Yes /No) | Trend Aligns (Yes / No) | Projected Value (mmHg) | Calculated Values (mmHg) | Difference (mmHg) | Acceptance Criteria (+/-4mmHg) |
| Scenario 1 Relatively Stable | | | | | | |
| 180 | Yes | Yes | 100.0 | 100.0 | 0 | Pass |
| Scenario 2 Fast Increase (> 4mmHg/min) | | | | | | |
| 90 | Yes | Yes | N/A | N/A | N/A | N/A |
| 120 | Yes | Yes | 87.5 | 87.5 | 0.0 | Pass |
| 150 | Yes | Yes | 88.5 | 88.6 | 0.1 | Pass |
| 180 | Yes | Yes | 93.4 | 93.4 | 0.0 | Pass |
| Scenario 3 Fast Decrease (> 4mmHg/min) | | | | | | |
| 90 | Yes | Yes | 85.7 | 85.5 | 0.2 | Pass |
| 120 | Yes | Yes | 75.5 | 75.4 | 0.1 | Pass |
| 150 | Yes | Yes | 71.6 | 71.6 | 0.0 | Pass |
| 180 | Yes | Yes | 68.1 | 68.0 | 0 | Pass |

Log File Data Re-Analysis

The results for this test are shown below, where MAPE is mean absolute percentage error.

Table 3: Relationship between Rate of Change of MAP and Projection Accuracy

| Change Condition | MAPE Values | | | Acceptance Criteria MAPE value must be 0-20% | Comparison |
|---|---|---|---|---|---|
| | Data Set 1 | Data Set 2 | Data Set 3 | | |
| **Across Range (60 - 110 mmHg)** | 3.3% | 6.6% | 4.4% | Pass | Highly Accurate |
| | | | | | |
| **MAP Increasing** | | | | | |
| Very fast | N/A | 18.2% | 9.3% | Pass | Good |
| Fast (2-4 mmHg) | N/A | 7.0% | 6.3% | Pass | Highly Accurate |
| Moderate (1-2 mmHg/min) | N/A | 6.40% | 4.5% | Pass | Highly Accurate |
| Flat/slow (0 - 1 mmHg/min) | N/A | 4.2% | 2.8% | Pass | Highly Accurate |
| **MAP Decreasing** | | | | | |
| Flat/slow (-1 to 0 mmHg/min) | 3.1% | 3.9% | 2.2% | Pass | Highly Accurate |
| Moderate (-2 to -1 mmHg/min) | 3.5% | 5.1% | 4.9% | Pass | Highly Accurate |
| Fast (-4 to -2 mmHg/min) | 2.2% | 8.2% | 9.2% | Pass | Highly Accurate |
| Very fast (< -4 mmHg/min) | 7.8% | 15.6% | 15.7% | Pass | Good |

The projection is for up to $T_{proj}$ (typically 2) minutes into the future.

The projection will show the implications of continuing the current trend. This will enable the user to make decisions about patient state and need for treatment.

The dataset for fitting will consist of the last 2 minutes of filtered $\widetilde{MAP}$ data points preceding the current time, ($t_{Now}$).

Use a linear fit to the data

$$y = A + Bx$$

Where $x$ = *time*, $y$ = *MAP*. Subscript $k$ denotes the values at time $t_k$. There are $n_k$ data points in the fitting period.
The sample estimates are $a$ (of $A$) and $b$ of ($B$).

The means are $\overline{x} = \frac{\sum x_k}{n_k}$ and $\overline{y} = \frac{\sum y_k}{n_k}$, and the standard deviations are

$$S_x = \sqrt{\frac{\sum_{i=1}^{n_k}(x_i-\overline{x})^2}{(n_k-1)}} \text{ and } S_y = \sqrt{\frac{\sum_{i=1}^{n_k}(y_i-\overline{y})^2}{(n_k-1)}}$$

**Slope and Intercept**

The slope estimate is $b = \frac{\sum_{i=1}^{n_k} x_i y_i - n_k \overline{x}\overline{y}}{\sum_{i=1}^{n_k} x_i^2 - n_k \overline{x}^2}$ and the intercept estimate is $a = \overline{y} - b\overline{x}$.

The residual standard deviation of y about the regression line is

$$S_{res} = \sqrt{\frac{(n_k - 1)(S_y^2 - b^2 S_x^2)}{(n_k - 2)}}$$

High values of $S_{res}$ may suggest that there is a poor fit to the data, and lack of a consistent trend. In the preferred embodiment we use a threshold of 1.5, although may of course be different in other implementations. If $S_{res}$ exceeds the threshold, the visual display of the projection may be suppressed.
Other methods such as the Kruskal Wallis test may be used.

**Confidence Interval of the Regression Line** (Altman and Gardner, 1988)

The confidence interval (CI) of the regression line is estimated to contain 95% of all lines through the current sample.
The estimated mean value of y for any $x$, say $x_0$, is

$$y_{fit} = a + bx_0$$

The standard error of $y_{fit}$ is

$$SE(y_{fit}) = S_{res}\sqrt{\frac{1}{n_k} + \frac{(x_0 - \overline{x})^2}{(n_k - 1)S_x^2}}$$

The $100(1 - \alpha)$% confidence interval for the population mean value of $\hat{y}$ at $x = x_0$ is

$$\hat{y} = y_{fit} \pm t_{1-\frac{\alpha}{2},n_k-2} SE(y_{fit})$$

Where $t_{1-\frac{\alpha}{2},n_k-2}$ is the t-value for a level of $1 - \frac{\alpha}{2}$ (eg 1-0.05/2 = 97.5/100) and $n_{k-2}$ is the degrees of freedom.

**Prediction Interval**

The uncertainty of $y_{fit}$ as a prediction for y - the "prediction interval" - is wider than the associated confidence interval.
Estimated standard deviation of individual values:

$$S_{pred} = S_{res} \sqrt{1 + \frac{1}{n_k} + \frac{(x_0 - \bar{x})^2}{(n_k - 1)S_x^2}}$$

The 100(1 - $\alpha$)% prediction interval is

$$\hat{y} = y_{fit} \pm t_{1-\frac{\alpha}{2}, n_{k-2}} S_{pred}$$

The value of $t_{1-\frac{\alpha}{2}, n_{k-2}}$ when $\alpha$ = 0.001 and $n_{k-2} \to \infty$ is 2.326.

The prediction $\hat{y}$ is taken as the projection of *MAP*. It consists of 3 values - low, fit and high. These values is computed at the current time $t_{Now}$, one minute ahead and $T_{proj}$ minutes ahead.

<u>User Interface (UI)</u>

The features of the user interface are preferably:

- Gives prominence to MAP as the primary parameter
- Highlights MAP compared to a clinician-set target range
- Includes multiple simultaneous timescales to aid the clinician in assessing the patient and deciding treatment:

  ◦ 20 minutes default MAP history adjustable to the whole procedure duration
  ◦ 5 minutes of recent trends in the casual cardiovascular parameters including CO, SVR and heart rate (HR)
  ◦ 2 minutes forward projection of MAP trend compared to targets

- Display of cumulative time in MAP risk ranges
- Ability to add treatment markers to the physiological trends in order to show the effects of different treatments

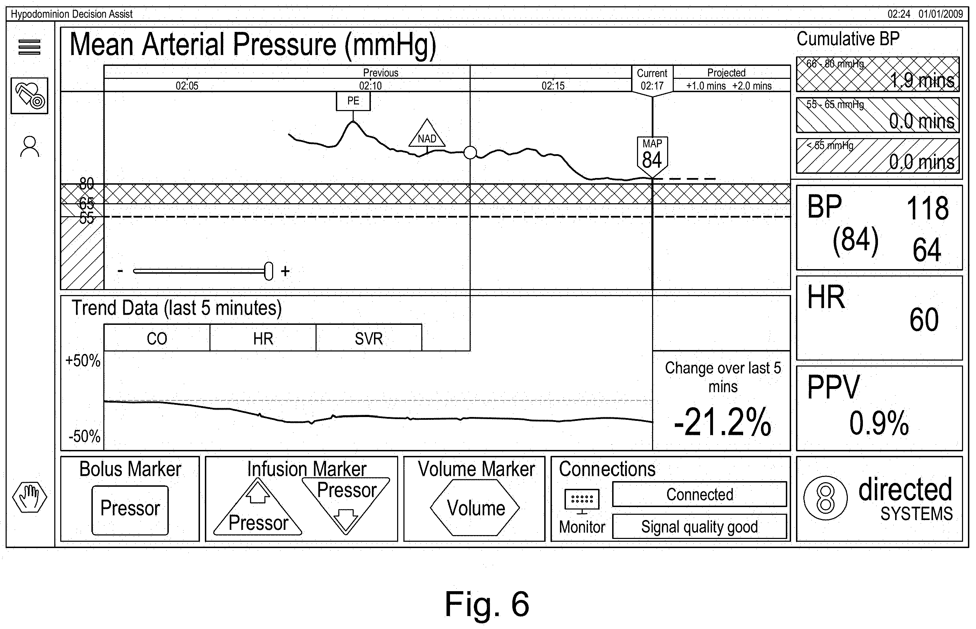

Figure 6 shows an example implementation of the UI, in this case showing data after 30 minutes of the patient's session.

In practice, the system is connected to the digital output ports of multiparameter patient vital signs monitors (for example from manufacturers such as Philips and GE) that are routinely used in the operating room. The vital signs monitor will also provide continuous arterial blood pressure waveform data and cardiovascular-related numeric parameters.

The medical user, an anaesthetist, has the option to set up a patient in the system by entering patient-specific information (height, weight, age) and define a target range for mean arterial blood pressure (MAP). The user will confirm that system is correctly communicating with the vital signs monitor that is connected to the patient.

In a routine operation, the system will track the vital signs of the patient. It may check that data are being acquired correctly and that the signals are of adequate quality and consistent, alerting the user with an appropriate action if not. The system will compute additional derived variables to help with its function and algorithms. For example, changes in cardiac output will be derived from the blood pressure waveform.

The system continually processes and displays, in graphical charts and numeric format, the data and derived variables in comparison with the user defined targets. It will detect and indicate to the user when blood pressure shown as MAP is below or is likely to fall below the target range. The system will allow the user to add labels to the graphic display chart to show the administration of vasopressors, as bolus or infused, and volume challenges.

Similarly, the system will indicate when MAP is above or likely to trend above the target range.

The intent of system is that with this information, the anaesthetist, or other medical professional, will make faster and more accurate assessment and treatment decisions regarding cardiovascular management. The anaesthetist may then implement these decisions manually with syringe boluses or vasopressors, or by changing the infusion rate of vasopressors, by volume challenges, or other means.

With reference to Figure 6, which is an example implementation of the system UI, the "Running Mode" screen displays MAP in the upper panel. Trend data (previous five minutes) for the selected parameter (CO/HR/SVR) are displayed in the lower panel. The percentage change over the last 5 minutes of the selected parameter is displayed immediately adjacent to the right of the trend display panel.

The default parameter displayed in the trend window is CO. The information in the other windows in this screen are described in subsequent sections of this user manual.

Target ranges for blood pressure may be set in the system. Upper and lower target levels for blood pressure can be set.

The upper pressure range limit can be varied between 70mmHg and 190mmHg. The lower pressure range limit can be varied between 40mmHg and 100mmHg.

The system's default setting for severe hypotension is <55mmHg.

The "Cumulative BP Thresholds" panel located in the upper right of the main "Running Mode" screen contains three icons that show the current target range and thresholds for moderate and severe hypotension. These are highlighted on the main MAP trace by colour.

Projection Line

When there is a consistent statistical trend (for example, when $S_{res}$ is below the threshold) in the mean arterial pressure (MAP), be it fast or slow, up or down, or flat, the trend and an uncertainty cone is projected forward from the current MAP value for the next 2 minutes. This is intended to help the medical professional decide whether the change is something they need to act on, and the user may use the MAP and recent changes parameters to decide what treatment they may take.

Marker labels can be entered on the screen display and data record to record/annotate specific events such as the bolus delivery of a drug.

Activation buttons for "Bolus Marker", "Infusion Marker" (up or down) and "Volume Marker" are located at the bottom left of the main display screen when in "Running Mode".

Adding a bolus marker to the display reveals a pop-up screen that includes a list of routinely used vasopressors (which may be pre-configured differently for different sites). The list specifies the formal name of the vasopressor, the standard colour of vials containing the drug and the abbreviated name that will be displayed on the drug label added to the timeline when the bolus marker is activated. The vasopressor administered can be selected from the menu displayed. Adding the bolus marker adds a triangular symbol with the 3-letter drug abbreviation, to the display screen as well as recording the event and time in the data record.

Adding an infusion marker to the display reveals a pop-up screen. Markers can be added to the screen display and data record for both increases and decreases in the infusion rate of drugs delivered to the patient during the blood pressure monitoring session.

As with the bolus marker, a list of routinely used vasopressors is presented on the screen. The list specifies the formal name of the vasopressor, the standard colour of vials containing the drug and the abbreviated name that will be displayed on the drug label added to the timeline when the marker is activated. Touching the "OK" button adds a marker to the display screen and records the event and time in the data record.

To add a marker signifying a volume challenge has been initiated, the user may press an icon that reveals a pop-up screen. Touching the "OK" adds a marker to the display screen and records the event and time in the data record.

**Alerts displayed on screen during use of HDA.**

If there has been a period of time (for example more than 30 seconds) with no signal being received, when a new blood pressure signal is detected by the system, the user will be alerted on the UI. Various status alerts may be displayed, for example: "Disconnected", "Connecting" and "Connected".

An alert may be displayed on the main screen if the quality of the blood pressure signal being received from the multiparameter vital signs monitor is such that system cannot compute accurate parameter values. If there are periods where the arterial pressure signal quality is too low to compute parameters (such as $\Delta CO$), the trend chart will include gaps.

References

Altman, D. G. and Gardner, M. J. (1988) 'Calculating confidence intervals for regression and correlation.', British medical journal (Clinical research ed.), 296(6631), pp. 1238-1242. doi: 10.1136/bmj.296.6634.1454.

Arulkumaran, N. et al. (2014) 'Cardiac complications associated with goal-directed therapy in high-risk surgical patients: A meta-analysis', British Journal of Anaesthesia, pp. 648-659. doi: 10.1093/bja/aet466.

Broch, O. et al. (2016a) 'Accuracy of Cardiac Output by Nine Different Pulse Contour Algorithms in Cardiac Surgery Patients: A Comparison with Transpulmonary Thermodilution', BioMed Research International, 2016. doi: 10.1155/2016/3468015.

Broch, O. et al. (2016b) 'Accuracy of Cardiac Output by Nine Different Pulse Contour Algorithms in Cardiac Surgery Patients: A Comparison with Transpulmonary Thermodilution', BioMed Research International, 2016. doi: 10.1155/2016/3468015.

Cecconi, M., Aya, H. D., et al. (2013) 'Changes in the mean systemic filling pressure during a fluid challenge in postsurgical intensive care patients.', Intensive care medicine, 39(7), pp. 1299-305. doi: 10.1007/s00134-013-2928-6.

Cecconi, M., Corredor, C., et al. (2013) 'Clinical review: Goal-directed therapy-what is the evidence in surgical patients? The effect on different risk groups.', Critical care (London, England), 17(2), p. 209. doi: 10.1186/cc11823.

Dimick, J. B. et al. (2004) 'Hospital costs associated with surgical complications: A report from the private-sector National Surgical Quality Improvement Program', Journal of the American College of Surgeons, 199(4), pp. 531-537. doi: 10.1016/j.jamcollsurg.2004.05.276.

Ebm, C. et al. (2014) 'A Cost-Effectiveness Analysis of Postoperative Goal-Directed Therapy for High-Risk Surgical Patients*', Critical Care Medicine, 42(5), pp. 1194-1203. doi: 10.1097/CCM.0000000000000164.

Fearon, K. C. H. et al. (2005) 'Enhanced recovery after surgery: A consensus review of clinical care for patients undergoing colonic resection', Clinical Nutrition, 24(3), pp. 466-477. doi: 10.1016/j.clnu.2005.02.002.

Futier, E. et al. (2017) 'Effect of Individualized vs Standard Blood Pressure Management Strategies on Postoperative Organ Dysfunction Among High-Risk Patients Undergoing Major Surgery', Jama, 318(14), p. 1346. doi: 10.1001/jama.2017.14172.

Ghaferi, A. A., Birkmeyer, J. D. and Dimick, J. B. (2009) 'Complications, Failure to Rescue, and Mortality With Major Inpatient Surgery in Medicare Patients', Annals of Surgery, 250(6), pp. 1029-1034. doi: 10.1097/SLA.0b013e3181-bef697.

Hamilton, M. A., Cecconi, M. and Rhodes, A. (2011) 'A systematic review and meta-analysis on the use of preemptive hemodynamic intervention to improve postoperative outcomes in moderate and high-risk surgical patients', Anesthesia and Analgesia, pp. 1392-1402. doi: 10.1213/ANE.0b013e3181eeaae5.

Hirsch, J. et al. (2015) 'Impact of intraoperative hypotension and blood pressure fluctuations on early postoperative delirium after non-cardiac surgery', British Journal of Anaesthesia, 115(3), pp. 418-426. doi: 10.1093/bja/aeu458.

Hsieh, J. K. et al. (2016) 'The Association between Mild Intraoperative Hypotension and Stroke in General Surgery Patients', Anesthesia and Analgesia, 123(4), pp. 933-939. doi: 10.1213/ANE.0000000000001526.

Khuri, S. F. et al. (2005) 'Determinants of long-term survival after major surgery and the adverse effect of postoperative complications', Ann Surg, 242(3), pp. 323-326.

Lee, J. M. et al. (2013) 'Effect of acute endotoxemia on analog estimates of mean systemic pressure', Journal of Critical Care. Elsevier B.V., 28(5), pp. 880.e9-880.e15. doi: 10.1016/j.jcrc.2013.04.007.

Liljestrand, G. and Zander, E. (1928) 'Vergleichende Bestimmungen des Minutenvolumens des Herzens beim Menschen mittels der Stickoxydulmethode und durch Blutdruckmessung', Zeitschrift für die gesamte experimentelle Medizin, 59(1), pp. 105-122. doi: 10.1007/BF02608853.

Maas, J. J. et al. (2012) 'Estimation of mean systemic filling pressure in postoperative cardiac surgery patients with three methods.', Intensive care medicine, 38(9), pp. 1452-60. doi: 10.1007/s00134-012-2586-0.

Maheshwari, K. et al. (2018) 'The relationship between ICU hypotension and in-hospital mortality and morbidity in septic patients', Intensive Care Medicine. Springer Berlin Heidelberg, 44(6), pp. 857-867. doi: 10.1007/s00134-018-5218-5.

McCormick, P. J. et al. (2016) 'Effectiveness of an Electronic Alert for Hypotension and Low Bispectral Index on 90-day Postoperative Mortality', Anesthesiology, 125(6), pp. 1113-1120. doi: 10.1097/ALN.0000000000001296.

Parkin, G. et al. (1994) 'Use of a mean systemic filling pressure analogue during the closed-loop control of fluid replacement in continuous hemodiafiltration.', Journal of critical care, 9(2), pp. 124-33. Available at: http://www.ncbi.nlm.nih.gov/pubmed/7920979.

Parkin, W. G. and Leaning, M. S. (2008) 'Therapeutic control of the circulation.', Journal of clinical monitoring and computing, 22, pp. 391-400. doi: 10.1007/s10877-008-9147-7.

Pearse, R. M. et al. (2006) 'Identification and characterisation of the high-risk surgical population in the United Kingdom.', Critical care (London, England), 10(3), p. R81. doi: 10.1186/cc4928.

Pellegrino, V. a et al. (2011) 'Computer based haemodynamic guidance system is effective and safe in management of postoperative cardiac surgery patients.', Anaesthesia and intensive care, 39(2), pp. 191-201. Available at: http://www.ncbi.nlm.nih.gov/pubmed/21485666.

Salmasi, V. et al. (2017) 'Relationship between Intraoperative Hypotension, Defined by Either Reduction from Baseline or Absolute Thresholds, and Acute Kidney and Myocardial Injury after Noncardiac Surgery', Anesthesiology, 126(1), pp. 47-65. doi: 10.1097/ALN.0000000000001432.

Scott, M. J. et al. (2015) 'Enhanced Recovery after Surgery (ERAS) for gastrointestinal surgery, part 1: Pathophysiological considerations', Acta Anaesthesiologica Scandinavica, pp. 1212-1231. doi: 10.1111/aas.12601.

Sondergaard, S. et al. (2012) 'High concordance between expert anaesthetists' actions and advice of decision support system in achieving oxygen delivery targets in high-risk surgery patients.', British journal of anaesthesia, 108(6), pp. 966-72. doi: 10.1093/bja/aes037.

Stapelfeldt, W. H. et al. (2017) 'The SLUScore: A novel method for detecting hazardous hypotension in adult patients undergoing noncardiac surgical procedures', Anesthesia and Analgesia, 124(4), pp. 1135-1152. doi:

10.1213/ANE.0000000000001797.

Sun, J. X., Reisner, A. T. and Mark, R. G. (2006) 'A signal abnormality index for arterial blood pressure waveforms', Computers in Cardiology.

Walsh, M. et al. (2013) 'Relationship between Intraoperative Mean Arterial Pressure and Clinical Outcomes after Noncardiac Surgery', Anesthesiology, 119(3), pp. 507-515. doi: 10.1097/ALN.0b013e3182a10e26.

Weiser, T. G. et al. (2008) 'An estimation of the global volume of surgery: a modelling strategy based on available data', The Lancet, 372(9633), pp. 139-144. doi: 10.1016/S0140-6736(08)60878-8.

Zong, W. et al. (2003) 'An open-source algorithm to detect onset of arterial blood pressure pulses', in Computers in Cardiology, 2003. doi: 10.1109/CIC.2003.1291140. Sun JX, Reisner AT, Saeed M, Heldt T, Mark RG. The cardiac output from blood Monge Garcia MI, Romero MG, Cano AG, Rhodes A, Grounds RM, Cecconi M. Impact of arterial load on the agreement between pulse pressure analysis and esophageal Doppler. Crit Care 2013; 17(3): R113.

Zhang J, Critchley LA, Huang L. Five algorithms that calculate cardiac output from the arterial waveform: a comparison with Doppler ultrasound. Br J Anaesth 2015;115: 392-402

Caillard A, Gayat E, Tantot A, Dubreuil G, M'Bakulu E, Madadaki C, Bart F, Bresson D, Froelich S, Mebazaa A, Vallée F. Comparison of cardiac output measured by oesophageal Doppler ultrasonography or pulse pressure contour wave analysis. Br J Anaesth 2015; 114: 893-900.

Atlas G, Berger J, Dhar S. Afterload assessment with or without central venous pressure: A preliminary clinical comparison. Cardiovasc Eng 2010; 10(4): 246-252

**Claims**

1. A system for determining relative changes in at least one parameter of cardiovascular function, the system comprising:

   an input for receiving arterial blood pressure waveform data; and
   signal processing apparatus, coupled to said input and configured to:

   detect a series of pulses from the arterial blood pressure waveform data;
   estimate at least one baseline value of nominal cardiac output for a first pulse using data from the first pulse and a second pulse within the detected series of pulses, wherein the first pulse is consecutive to the second pulse; filter data from the arterial blood pressure waveform data with a time-moving mean filter;
   estimate at least one value of nominal cardiac output from the filtered data for a time period comprising a sequence of a plurality, n, of pulses wherein there is a predetermined and constant time period between the first pulse of the detected series of pulses and the sequence of n pulses;
   for each of the nominal cardiac output values, calculate the relative change of the respective nominal cardiac output value based on the difference between the respective nominal cardiac output value and the baseline nominal cardiac output value.

2. A system according to claim 1, wherein the signal processing apparatus is further configured to calculate the relative change in systemic vascular resistance using the relative change in nominal cardiac output; and
   optionally wherein the relative change in systemic vascular resistance is calculated by dividing a time-moving mean filtered of mean arterial blood pressure by the relative change in nominal cardiac output.

3. A system according to claim 2, wherein the relative change in systemic vascular resistance is calculated by:

   calculating a baseline total systemic vascular resistance for a first pulse;
   calculating total systemic vascular resistance for a plurality of pulses within a time period comprising a sequence of n pulses in the arterial blood pressure waveform data; and
   for each of the total systemic vascular resistance values, calculating the relative change of the respective total systemic vascular resistance based on the difference between the respective total systemic vascular resistance value and the baseline total systemic vascular resistance value; and

   optionally wherein total systemic vascular resistance is calculated by dividing mean arterial blood pressure by nominal cardiac output.

4. A system according to any preceding claim, wherein the signal processing apparatus is further configured to calculate the relative change in venous return driving pressure using nominal cardiac output and mean arterial blood pressure;

and

optionally wherein the relative change in venous return driving pressure is calculated from the relative change in cardiac output and a change in mean arterial pressure.

5. A system according to any preceding claim, wherein the system further comprises a memory configured to store input arterial blood pressure waveform data and nominal cardiac output data.

6. A system according to any preceding claim, wherein the signal processing apparatus is further configured to determine if at least one of nominal cardiac output, systemic vascular resistance, or venous return driving pressure is within a predetermined range; and

   optionally wherein the system is configured to alert a user if said at least one of nominal cardiac output, systemic vascular resistance, or venous return driving pressure is outside the predetermined range.

7. A system according to claim 6, wherein the signal processing apparatus is further configured to assign a signal abnormality index value to a pulse in the sequence of $n$ pulses dependent upon the outcome of said determination, wherein the pulse corresponds to a pulse for which said at least one of nominal cardiac output, systemic vascular resistance, or venous return driving pressure is outside the predetermined range; and/or

   wherein the signal processing apparatus is further configured to determine a length of time at least one of relative change in nominal cardiac output, systemic vascular resistance, or venous return driving pressure is outside the predetermined range.

8. A system according to any preceding claim, wherein the signal processing apparatus is further configured to extrapolate said at least one of nominal cardiac output, systemic vascular resistance, or venous return driving pressure to a future time.

9. A system according to claim 8, wherein the signal processing apparatus is configured to determine if a statistical trend is present in said at least one of nominal cardiac output, systemic vascular resistance, or venous return driving pressure, and wherein the signal processing apparatus is configured to output the extrapolated nominal cardiac output, systemic vascular resistance, or venous return driving pressure at a future time dependent upon said outcome of determination of statistical trend.

10. A system according to claim 8 or 9, wherein the signal processing apparatus is configured to extrapolate the at least one of nominal cardiac output, systemic vascular resistance, or venous return driving pressure at a future time using a linear regression technique; and

    optionally wherein the signal processing apparatus is configured to determine if a statistical trend is present in the at least one of nominal cardiac output, systemic vascular resistance, or venous return driving pressure by calculating the number of standard deviations of nominal cardiac output, systemic vascular resistance, or venous return driving pressure about a regression line and determining if the number of standard deviations is greater than a predetermined threshold.

11. A system according to claim 8, 9, or 10, wherein the signal processing apparatus is configured to extrapolate the at least one of nominal cardiac output, systemic vascular resistance, or venous return driving pressure at a future time if a treatment is administered.

12. A system according to any of claims 8 to 11, wherein the system is configured to alert a user if said extrapolated at least one of nominal cardiac output, systemic vascular resistance, or venous return driving pressure at a future time is outside the predetermined range.

13. A system according to any preceding claim, further comprising a user interface configured to display the relative change in at least one of nominal cardiac output, systemic vascular resistance, or venous return driving pressure; and/or

    wherein the signal processing apparatus is configured to remove artefacts from the arterial blood pressure waveform data.

14. A system according to any preceding claim, wherein the system is configured to selectively output the calculated relative change of nominal cardiac output, systemic vascular resistance, or venous return driving pressure dependent upon the signal quality of the arterial blood pressure waveform data received from the input, and wherein the system outputs the calculated relative change of nominal cardiac output, systemic vascular resistance, or venous return driving pressure only when the signal quality of the arterial blood pressure waveform data is above a threshold value.

15. A computer-implemented method of determining relative changes in at least one parameter of cardiovascular function, the method comprising:

receiving arterial blood pressure waveform data;
detecting a series of pulses from the arterial blood pressure waveform data;
estimating at least one baseline value of nominal cardiac output for a first pulse using data from the first pulse and a second pulse within the detected series of pulses, wherein the first pulse is consecutive to the second pulse;
filtering data from the arterial blood pressure waveform data with a time-moving mean filter;
estimating at least one value of nominal cardiac output from the filtered data for a time period comprising a sequence of a plurality, n, of pulses wherein there is a predetermined and constant time period between the first pulse of the detected series of pulses and the sequence of *n* pulses; and
for each of the nominal cardiac output values, calculating the relative change of the respective nominal cardiac output value based on the difference between the respective nominal cardiac output value and the baseline nominal cardiac output value.

**Patentansprüche**

1. System zum Bestimmen relativer Veränderungen an mindestens einem Parameter kardiovaskulärer Funktion, wobei das System Folgendes umfasst:

einen Eingang zum Empfangen von Kurvenverlaufsdaten arteriellen Blutdrucks; und
eine Signalverarbeitungsvorrichtung, an den Eingang gekoppelt und dazu konfiguriert:

eine Reihe von Impulsen aus den Kurvenverlaufsdaten arteriellen Blutdrucks zu erkennen;
mindestens einen Ausgangswert des Soll-Herzzeitvolumens für einen ersten Impuls unter Verwendung von Daten aus dem ersten Impuls und einem zweiten Impuls innerhalb der erkannten Reihe von Impulsen zu schätzen, wobei der erste Impuls mit dem zweiten Impuls aufeinanderfolgend ist;
Daten aus den Kurvenverlaufsdaten arteriellen Blutdrucks mit einem zeitlich gleitenden Mittelwertfilter zu filtern;
mindestens einen Wert des Soll-Herzzeitvolumens aus den gefilterten Daten für eine Zeitdauer zu schätzen, die eine Sequenz einer Vielzahl, n, von Impulsen umfasst,
wobei eine vorbestimmte und konstante Zeitdauer zwischen dem ersten Impuls der erkannten Reihe von Impulsen und der Sequenz von n Impulsen liegt;
für jeden der Soll-Herzzeitvolumenwerte die relative Veränderung des jeweiligen Soll-Herzzeitvolumenwerts basierend auf der Differenz zwischen dem jeweiligen Soll-Herzzeitvolumenwert und dem Soll-Herzzeitvolumenausgangswerts zu berechnen.

2. System nach Anspruch 1, wobei die Signalverarbeitungsvorrichtung ferner dazu konfiguriert ist, die relative Veränderung des systemischen Gefäßwiderstands unter Verwendung der relativen Veränderung des Soll-Herzzeitvolumens zu berechnen; und
optional wobei die relative Veränderung des systemischen Gefäßwiderstands berechnet wird, indem ein zeitlich gleitender gefilterter Mittelwert des mittleren arteriellen Blutdrucks durch die relative Veränderung des Soll-Herzzeitvolumens geteilt wird.

3. System nach Anspruch 2, wobei die relative Veränderung des systemischen Gefäßwiderstands berechnet wird durch:

Berechnen einer Basislinie des gesamten systemischen Gefäßwiderstands für einen ersten Impuls;
Berechnen des gesamten systemischen Gefäßwiderstands für eine Vielzahl von Impulsen innerhalb einer Zeitdauer umfassend eine Sequenz von n Impulsen in den Kurvenverlaufsdaten arteriellen Blutdrucks; und
für jeden der Werte des gesamten systemischen Gefäßwiderstands, Berechnen der relativen Veränderung des jeweiligen gesamten systemischen Gefäßwiderstands basierend auf der Differenz zwischen dem jeweiligen Wert des gesamten systemischen Gefäßwiderstands und dem Ausgangswert des gesamten systemischen Gefäßwiderstands; und
optional wobei der gesamte systemische Gefäßwiderstand berechnet wird durch Teilen des mittleren arteriellen Blutdrucks durch das Soll-Herzzeitvolumen.

4. System nach einem vorhergehenden Anspruch, wobei die Signalverarbeitungsvorrichtung ferner dazu konfiguriert ist, die relative Veränderung des Antriebsdrucks des venösen Rückflusses unter Verwendung des Soll-Herzzeitvolumens und des mittleren arteriellen Blutdrucks zu berechnen; und
optional wobei die relative Veränderung des Antriebsdrucks des venösen Rückflusses aus der relativen Veränderung des Herzzeitvolumens und einer Veränderung des mittleren arteriellen Drucks berechnet wird.

5. System nach einem vorhergehenden Anspruch, wobei das System ferner einen Speicher umfasst, der dazu konfiguriert ist, eingegebene Kurvenverlaufsdaten arteriellen Blutdrucks und Soll-Herzzeitvolumendaten zu speichern.

6. System nach einem vorhergehenden Anspruch, wobei die Signalverarbeitungsvorrichtung ferner dazu konfiguriert ist, zu bestimmen, ob mindestens eines von Soll-Herzzeitvolumen, systemischem Gefäßwiderstand oder Antriebsdruck des venösen Rückflusses innerhalb eines vorbestimmten Bereichs liegt; und
optional wobei das System dazu konfiguriert ist, einen Benutzer zu alarmieren, wenn das mindestens eine von Soll-Herzzeitvolumen, systemischem Gefäßwiderstand oder Antriebsdruck des venösen Rückflusses außerhalb des vorbestimmten Bereichs liegt.

7. System nach Anspruch 6, wobei die Signalverarbeitungsvorrichtung ferner dazu konfiguriert ist, einem Impuls in der Sequenz von n Impulsen einen Signalauffälligkeitsindexwert zuzuordnen abhängig von dem Ergebnis der Bestimmung, wobei der Impuls einem Impuls entspricht, für den das mindestens eine von Soll-Herzzeitvolumen, systemischem Gefäßwiderstand oder Antriebsdruck des venösen Rückflusses außerhalb des vorbestimmten Bereichs liegt; und/oder
wobei die Signalverarbeitungsvorrichtung ferner dazu konfiguriert ist, eine Zeitdauer zu bestimmen, für die mindestens eines von relativer Veränderung des Soll-Herzzeitvolumens, systemischen Gefäßwiderstands oder Antriebsdrucks des venösen Rückflusses außerhalb des vorbestimmten Bereichs liegt.

8. System nach einem vorhergehenden Anspruch, wobei die Signalverarbeitungsvorrichtung ferner dazu konfiguriert ist, das mindestens eine von Soll-Herzzeitvolumen, systemischem Gefäßwiderstand oder Antriebsdruck des venösen Rückflusses zu einem zukünftigen Zeitpunkt zu extrapolieren.

9. System nach Anspruch 8, wobei die Signalverarbeitungsvorrichtung dazu konfiguriert ist, zu bestimmen, ob bei dem mindestens einen von Soll-Herzzeitvolumen, systemischem Gefäßwiderstand oder Antriebsdruck des venösen Rückflusses ein statistischer Trend vorliegt, und wobei die Signalverarbeitungsvorrichtung dazu konfiguriert ist, das/den extrapolierte(n) Soll-Herzzeitvolumen, systemischen Gefäßwiderstand oder Antriebsdruck des venösen Rückflusses zu einem zukünftigen Zeitpunkt abhängig von dem Ergebnis einer Bestimmung eines statistischen Trends auszugeben.

10. System nach Anspruch 8 oder 9, wobei die Signalverarbeitungsvorrichtung dazu konfiguriert ist, das mindestens eine von Soll-Herzzeitvolumen, systemischem Gefäßwiderstand oder Antriebsdruck des venösen Rückflusses zu einem zukünftigen Zeitpunkt unter Verwendung einer Technik linearer Regression zu extrapolieren; und
optional wobei die Signalverarbeitungsvorrichtung dazu konfiguriert ist, zu bestimmen, ob bei dem mindestens einen von Soll-Herzzeitvolumen, systemischem Gefäßwiderstand oder Antriebsdruck des venösen Rückflusses ein statistischer Trend vorliegt durch Berechnen der Anzahl von Standardabweichungen von Soll-Herzzeitvolumen, systemischem Gefäßwiderstand oder Antriebsdruck des venösen Rückflusses um eine Regressionsgerade und Bestimmen, ob die Anzahl von Standardabweichungen größer ist als ein vorbestimmter Schwellenwert.

11. System nach Anspruch 8, 9 oder 10, wobei die Signalverarbeitungsvorrichtung dazu konfiguriert ist, das mindestens eine von Soll-Herzzeitvolumen, systemischem Gefäßwiderstand oder Antriebsdruck des venösen Rückflusses zu einem zukünftigen Zeitpunkt zu extrapolieren, wenn eine Behandlung vorgenommen wird.

12. System nach einem der Ansprüche 8 bis 11, wobei das System dazu konfiguriert ist, einen Benutzer zu alarmieren, wenn das extrapolierte mindestens eine von Soll-Herzzeitvolumen, systemischem Gefäßwiderstand oder Antriebsdruck des venösen Rückflusses zu einem zukünftigen Zeitpunkt außerhalb des vorbestimmten Bereichs liegt.

13. System nach einem vorhergehenden Anspruch, ferner umfassend eine Benutzerschnittstelle, die dazu konfiguriert ist, die relative Veränderung von mindestens einem von Soll-Herzzeitvolumen, systemischem Gefäßwiderstand oder Antriebsdruck des venösen Rückflusses anzuzeigen; und/oder
wobei die Signalverarbeitungsvorrichtung dazu konfiguriert ist, Artefakte aus den Kurvenverlaufsdaten arteriellen Blutdrucks zu entfernen.

14. System nach einem vorhergehenden Anspruch, wobei das System dazu konfiguriert ist, die berechnete relative Veränderung des Soll-Herzzeitvolumens, systemischen Gefäßwiderstands oder Antriebsdrucks des venösen Rückflusses selektiv auszugeben, abhängig von der Signalqualität der von dem Eingang empfangenen Kurvenverlaufsdaten arteriellen Blutdrucks, und wobei das System die berechnete relative Veränderung des Soll-Herzzeitvolumens, systemischen Gefäßwiderstands oder Antriebsdrucks des venösen Rückflusses nur ausgibt, wenn die Signalqualität der Kurvenverlaufsdaten arteriellen Blutdrucks über einem Schwellenwert liegt.

15. Computerimplementiertes Verfahren zum Bestimmen relativer Veränderungen an mindestens einem Parameter kardiovaskulärer Funktion, wobei das Verfahren Folgendes umfasst:

Empfangen von Kurvenverlaufsdaten arteriellen Blutdrucks;
Erkennen einer Reihe von Impulsen aus den Kurvenverlaufsdaten arteriellen Blutdrucks;
Schätzen mindestens eines Ausgangswerts des Soll-Herzzeitvolumens für einen ersten Impuls unter Verwendung von Daten aus dem ersten Impuls und einem zweiten Impuls innerhalb der erkannten Reihe von Impulsen, wobei der erste Impuls mit dem zweiten Impuls aufeinanderfolgend ist;
Filtern von Daten aus den Kurvenverlaufsdaten arteriellen Blutdrucks mit einem zeitlich gleitenden Mittelwertfilter;
Schätzen mindestens eines Werts des Soll-Herzzeitvolumens aus den gefilterten Daten für eine Zeitdauer, die eine Sequenz einer Vielzahl, n, von Impulsen umfasst,
wobei eine vorbestimmte und konstante Zeitdauer zwischen dem ersten Impuls der erkannten Reihe von Impulsen und der Sequenz von n Impulsen liegt; und
für jeden der Soll-Herzzeitvolumenwerte, Berechnen der relativen Veränderung des jeweiligen Soll-Herzzeitvolumenwerts basierend auf der Differenz zwischen dem jeweiligen Soll-Herzzeitvolumenwert und dem Soll-Herzzeitvolumenausgangswerts.

## Revendications

1. Système permettant de déterminer des changements relatifs d'au moins un paramètre de la fonction cardiovasculaire, le système comprenant :

une entrée pour recevoir des données de forme d'onde de pression sanguine artérielle ; et
un appareil de traitement de signaux, couplé à ladite entrée et configuré pour :

détecter une série d'impulsions à partir des données de forme d'onde de pression sanguine artérielle ;
estimer au moins une valeur de base du débit cardiaque nominal pour une première impulsion en utilisant des données de la première impulsion et d'une deuxième impulsion dans la série d'impulsions détectée, dans lequel la première impulsion est consécutive à la deuxième impulsion ;
filtrer des données à partir des données de forme d'onde de pression sanguine artérielle en utilisant un filtre à moyenne mobile dans le temps ;
estimer au moins une valeur du débit cardiaque nominal à partir des données filtrées pour une période de temps comprenant une séquence d'une pluralité, n, d'impulsions,
dans lequel il existe une période de temps prédéterminée et constante entre la première impulsion de la série d'impulsions détectée et la séquence de n impulsions ;
pour chacune des valeurs de débit cardiaque nominal, calculer le changement relatif de la valeur respective de débit cardiaque nominal sur la base de la différence entre la valeur respective de débit cardiaque nominal et la valeur de base de débit cardiaque nominal.

2. Système selon la revendication 1, dans lequel l'appareil de traitement de signaux est en outre configuré pour calculer le changement relatif de la résistance vasculaire systémique en utilisant le changement relatif du débit cardiaque nominal ; et
facultativement, dans lequel le changement relatif de la résistance vasculaire systémique est calculé en divisant une moyenne mobile dans le temps filtrée de la pression sanguine artérielle moyenne par le changement relatif du débit cardiaque nominal.

**3.** Système selon la revendication 2, dans lequel le changement relatif de la résistance vasculaire systémique est calculé :

en calculant une résistance vasculaire systémique totale de base pour une première impulsion ;
en calculant la résistance vasculaire systémique totale pour une pluralité d'impulsions dans une période de temps comprenant une séquence de n impulsions dans les données de forme d'onde de pression sanguine artérielle ; et pour chacune des valeurs de résistance vasculaire systémique totale, en calculant le changement relatif de la résistance vasculaire systémique totale respective sur la base de la différence entre la valeur de résistance vasculaire systémique totale respective et la valeur de résistance vasculaire systémique totale de base ; et facultativement, dans lequel la résistance vasculaire systémique totale est calculée en divisant la pression sanguine artérielle moyenne par le débit cardiaque nominal.

**4.** Système selon l'une quelconque des revendications précédentes, dans lequel l'appareil de traitement de signaux est en outre configuré pour calculer le changement relatif de la pression d'entraînement de retour veineux en utilisant le débit cardiaque nominal et la pression sanguine artérielle moyenne ; et facultativement, dans lequel le changement relatif de la pression d'entraînement de retour veineux est calculé à partir du changement relatif du débit cardiaque et d'un changement de la pression artérielle moyenne.

**5.** Système selon l'une quelconque des revendications précédentes, dans lequel le système comprend en outre une mémoire configurée pour stocker des données de forme d'onde de pression sanguine artérielle d'entrée et des données de débit cardiaque nominal.

**6.** Système selon l'une quelconque des revendications précédentes, dans lequel l'appareil de traitement de signaux est en outre configuré pour déterminer si au moins l'un du débit cardiaque nominal, de la résistance vasculaire systémique ou de la pression d'entraînement de retour veineux se situe ou ne se situe pas dans une plage prédéterminée ; et facultativement, dans lequel le système est configuré pour alerter un utilisateur si au moins l'un du débit cardiaque nominal, de la résistance vasculaire systémique ou de la pression d'entraînement de retour veineux se situe en dehors de la plage prédéterminée.

**7.** Système selon la revendication 6, dans lequel l'appareil de traitement de signaux est en outre configuré pour attribuer une valeur d'indice d'anomalie de signal à une impulsion dans la séquence de n impulsions en fonction du résultat de ladite détermination, dans lequel l'impulsion correspond à une impulsion pour laquelle ledit au moins un du débit cardiaque nominal, de la résistance vasculaire systémique ou de la pression d'entraînement de retour veineux est en dehors de la plage prédéterminée ; et/ou dans lequel l'appareil de traitement de signaux est en outre configuré pour déterminer une durée pendant laquelle au moins l'un du changement relatif du débit cardiaque nominal, de la résistance vasculaire systémique ou de la pression d'entraînement de retour veineux est en dehors de la plage prédéterminée.

**8.** Système selon l'une quelconque des revendications précédentes, dans lequel l'appareil de traitement de signaux est en outre configuré pour extrapoler ledit au moins un du débit cardiaque nominal, de la résistance vasculaire systémique ou de la pression d'entraînement de retour veineux à un moment futur.

**9.** Système selon la revendication 8, dans lequel l'appareil de traitement de signaux est configuré pour déterminer si une tendance statistique est ou n'est pas présente dans ledit au moins un du débit cardiaque nominal, de la résistance vasculaire systémique ou de la pression d'entraînement de retour veineux, et dans lequel l'appareil de traitement de signaux est configuré pour émettre en sortie le débit cardiaque nominal extrapolé, la résistance vasculaire systémique ou la pression d'entraînement de retour veineux à un moment futur dépendant dudit résultat de détermination de tendance statistique.

**10.** Système selon la revendication 8 ou 9, dans lequel l'appareil de traitement de signaux est configuré pour extrapoler ledit au moins un du débit cardiaque nominal, de la résistance vasculaire systémique ou de la pression d'entraînement de retour veineux à un moment futur en utilisant une technique de régression linéaire ; et facultativement, dans lequel l'appareil de traitement de signaux est configuré pour déterminer si une tendance statistique est ou n'est pas présente dans ledit au moins un du débit cardiaque nominal, de la résistance vasculaire systémique ou de la pression d'entraînement de retour veineux en calculant le nombre d'écarts-types du débit cardiaque nominal, de la résistance vasculaire systémique ou de la pression d'entraînement de retour veineux autour d'une ligne de régression et en déterminant si le nombre d'écarts-types est ou n'est pas supérieur à un seuil prédéterminé.

11. Système selon la revendication 8, 9, ou 10, dans lequel l'appareil de traitement de signaux est configuré pour extrapoler au moins ledit au moins un du débit cardiaque nominal, de la résistance vasculaire systémique, ou de la pression d'entraînement de retour veineux, à un moment futur si un traitement est administré.

12. Système selon l'une quelconque des revendications 8 à 11, dans lequel le système est configuré pour alerter un utilisateur si ledit au moins un élément extrapolé parmi le débit cardiaque nominal, la résistance vasculaire systémique ou la pression d'entraînement de retour veineux à un moment futur est en dehors de la plage prédéterminée.

13. Système selon l'une quelconque des revendications précédentes, comprenant en outre une interface utilisateur configurée pour afficher le changement relatif d'au moins l'un du débit cardiaque nominal, de la résistance vasculaire systémique, ou de la pression d'entraînement de retour veineux ; et/ou
dans lequel l'appareil de traitement de signaux est configuré pour supprimer des artéfacts des données de forme d'onde de pression sanguine artérielle.

14. Système selon l'une quelconque des revendications précédentes, dans lequel le système est configuré pour émettre en sortie sélectivement le changement relatif calculé du débit cardiaque nominal, de la résistance vasculaire systémique ou de la pression d'entraînement de retour veineux en fonction de la qualité de signal des données de forme d'onde de pression sanguine artérielle reçues de l'entrée, et dans lequel le système émet en sortie le changement relatif calculé du débit cardiaque nominal, de la résistance vasculaire systémique ou de la pression d'entraînement de retour veineux uniquement lorsque la qualité de signal des données de forme d'onde de pression sanguine artérielle est supérieure à une valeur de seuil.

15. Procédé mis en œuvre par ordinateur pour déterminer des changements relatifs d'au moins un paramètre de la fonction cardiovasculaire, le procédé comprenant :

la réception de données de forme d'onde de pression sanguine artérielle ;
la détection d'une série d'impulsions à partir des données de forme d'onde de pression sanguine artérielle ;
l'estimation d'au moins une valeur de base du débit cardiaque nominal pour une première impulsion en utilisant des données de la première impulsion et d'une deuxième impulsion dans la série d'impulsions détectée, dans lequel la première impulsion est consécutive à la deuxième impulsion ;
filtrer des données à partir des données de forme d'onde de pression sanguine artérielle avec un filtre à moyenne mobile dans le temps ;
l'estimation d'au moins une valeur du débit cardiaque nominal à partir des données filtrées pour une période de temps comprenant une séquence d'une pluralité, n, d'impulsions,
dans lequel il existe une période de temps prédéterminée et constante entre la première impulsion de la série d'impulsions détectée et la séquence de n impulsions ; et
pour chacune des valeurs du débit cardiaque nominal, calculer le changement relatif de la valeur respective du débit cardiaque nominal sur la base de la différence entre la valeur respective du débit cardiaque nominal et la valeur de base du débit cardiaque nominal.

Fig. 1

Fig. 2

**Layer 1**

User Interface

Logging

Physiological Signals & Derived Data

**Layer 2**

Physiological Data Processing

Signal Processing and Signal Quality Check

Configuration Commands

Raw Physiological Signals

Raw Physiological Signals

**Layer 3**

Device Drivers

Fig. 3

```
                    ┌──────────────┐
          ┌────────▶│    Beat      │
          │         │  detection   │
          │         └──────┬───────┘
          │                │
   ABP    │                ▼
          │         ┌──────────────┐
          └────────▶│   Feature    │
                    │  Extraction  │
                    └──────┬───────┘
```

Abnormality Criterion 1

⋮

Abnormality Criterion n

Logical OR

→ SAI

## Fig. 4

ABP
$x_n$ → Low-pass filter → $y_n$ → Slope sum function → $z_n$ → Decision rule → $t_{onset}(0), t_{onset}(1), ...$

## Fig. 5

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011080190 A1

- EP 2563213 A


**Non-patent literature cited in the description**

- **ALTMAN, D. G.** ; **GARDNER, M. J.** Calculating confidence intervals for regression and correlation.. *British medical journal*, 1988, vol. 296 (6631), 1238-1242
- **ARULKUMARAN, N. et al.** Cardiac complications associated with goal-directed therapy in high-risk surgical patients: A meta-analysis. *British Journal of Anaesthesia*, 2014, 648-659
- **BROCH, O. et al.** Accuracy of Cardiac Output by Nine Different Pulse Contour Algorithms in Cardiac Surgery Patients: A Comparison with Transpulmonary Thermodilution. *BioMed Research International*, 2016
- **CECCONI, M** ; **AYA, H. D. et al.** Changes in the mean systemic filling pressure during a fluid challenge in postsurgical intensive care patients. *Intensive care medicine*, 2013, vol. 39 (7), 1299-305
- **CECCONI, M** ; **CORREDOR, C et al.** Clinical review: Goal-directed therapy-what is the evidence in surgical patients? The effect on different risk groups.. *Critical care*, 2013, vol. 17 (2), 209
- **DIMICK, J. B. et al.** Hospital costs associated with surgical complications: A report from the private-sector National Surgical Quality Improvement Program. *Journal of the American College of Surgeons*, 2004, vol. 199 (4), 531-537
- **EBM, C. et al.** A Cost-Effectiveness Analysis of Postoperative Goal-Directed Therapy for High-Risk Surgical Patients. *Critical Care Medicine*, 2014, vol. 42 (5), 1194-1203
- **FEARON, K. C. H. et al.** Enhanced recovery after surgery: A consensus review of clinical care for patients undergoing colonic resection. *Clinical Nutrition*, 2005, vol. 24 (3), 466-477
- **FUTIER, E. et al.** Effect of Individualized vs Standard Blood Pressure Management Strategies on Postoperative Organ Dysfunction Among High-Risk Patients Undergoing Major Surgery. *Jama*, 2017, vol. 318 (14), 1346
- **GHAFERI, A. A.** ; **BIRKMEYER, J. D.** ; **DIMICK, J. B.** Complications, Failure to Rescue, and Mortality With Major Inpatient Surgery in Medicare Patients. *Annals of Surgery*, 2009, vol. 250 (6), 1029-1034

- **HAMILTON, M. A** ; **CECCONI, M.** ; **RHODES, A.** A systematic review and meta-analysis on the use of preemptive hemodynamic intervention to improve postoperative outcomes in moderate and high-risk surgical patients. *Anesthesia and Analgesia*, 2011, 1392-1402
- **HIRSCH, J. et al.** Impact of intraoperative hypotension and blood pressure fluctuations on early postoperative delirium after non-cardiac surgery. *British Journal of Anaesthesia,*, 2015, vol. 115 (3), 418-426
- **HSIEH, J. K. et al.** The Association between Mild Intraoperative Hypotension and Stroke in General Surgery Patients. *Anesthesia and Analgesia*, 2016, vol. 123 (4), 933-939
- **KHURI, S. F. et al.** Determinants of long-term survival after major surgery and the adverse effect of postoperative complications. *Ann Surg*, 2005, vol. 242 (3), 323-326
- Effect of acute endotoxemia on analog estimates of mean systemic pressure. **LEE, J. M. et al.** Journal of Critical Care.. Elsevier B.V., 2013, vol. 28, 9-15
- **LILJESTRAND, G.** ; **ZANDER, E.** Vergleichende Bestimmungen des Minutenvolumens des Herzens beim Menschen mittels der Stickoxydulmethode und durch Blutdruckmessung. *Zeitschrift für die gesamte experimentelle Medizin*, 1928, vol. 59 (1), 105-122
- **MAAS, J. J. et al.** Estimation of mean systemic filling pressure in postoperative cardiac surgery patients with three methods. *Intensive care medicine*, 2012, vol. 38 (9), 1452-60
- The relationship between ICU hypotension and in-hospital mortality and morbidity in septic patients. **MAHESHWARI, K. et al.** Intensive Care Medicine. Springer, 2018, vol. 44, 857-867
- **MCCORMICK, P. J. et al.** Effectiveness of an Electronic Alert for Hypotension and Low Bispectral Index on 90-day Postoperative Mortality. *Anesthesiology*, 2016, vol. 125 (6), 1113-1120

- **PARKIN, G. et al.** Use of a mean systemic filling pressure analogue during the closed-loop control of fluid replacement in continuous hemodiafiltration. *Journal of critical care*, 1994, vol. 9 (2), 124-33, http://www.ncbi.nlm.nih.gov/pubmed/7920979
- **PARKIN, W. G.** ; **LEANING, M. S.** Therapeutic control of the circulation. *Journal of clinical monitoring and computing*, 2008, vol. 22, 391-400
- **PEARSE, R. M. et al.** Identification and characterisation of the high-risk surgical population in the United Kingdom.. *Critical care*, 2006, vol. 10 (3), R81
- **PELLEGRINO, V. A et al.** Computer based haemodynamic guidance system is effective and safe in management of postoperative cardiac surgery patients.. *Anaesthesia and intensive care*, 2011, vol. 39 (2), 191-201, http://www.ncbi.nlm.nih.gov/-pubmed/21485666.
- **SALMASI, V. et al.** Relationship between Intraoperative Hypotension, Defined by Either Reduction from Baseline or Absolute Thresholds, and Acute Kidney and Myocardial Injury after Noncardiac Surgery. *Anesthesiology*, 2017, vol. 126 (1), 47-65
- **SCOTT, M. J. et al.** Enhanced Recovery after Surgery (ERAS) for gastrointestinal surgery, part 1: Pathophysiological considerations. *Acta Anaesthesiologica Scandinavica,*, 2015, 1212-1231
- **SONDERGAARD, S. et al.** High concordance between expert anaesthetists' actions and advice of decision support system in achieving oxygen delivery targets in high-risk surgery patients. *British journal of anaesthesia*, 2012, vol. 108 (6), 966-72
- **STAPELFELDT, W. H. et al.** The SLUScore: A novel method for detecting hazardous hypotension in adult patients undergoing noncardiac surgical procedures. *Anesthesia and Analgesia*, 2017, vol. 124 (4), 1135-1152
- **SUN, J. X.** ; **REISNER, A. T.** ; **MARK, R. G**. A signal abnormality index for arterial blood pressure waveforms. *Computers in Cardiology.*, 2006
- **WALSH, M. et al.** Relationship between Intraoperative Mean Arterial Pressure and Clinical Outcomes after Noncardiac Surgery. *Anesthesiology*, 2013, vol. 119 (3), 507-515
- **WEISER, T. G. et al.** An estimation of the global volume of surgery: a modelling strategy based on available data. *The Lancet*, 2008, vol. 372 (9633), 139-144
- **ZONG, W. et al.** An open-source algorithm to detect onset of arterial blood pressure pulses. *Computers in Cardiology*, 2003
- **SUN JX** ; **REISNER AT** ; **SAEED M** ; **HELDT T** ; **MARK RG.** *The cardiac output from blood Monge*
- **GARCIA MI** ; **ROMERO MG** ; **CANO AG** ; **RHODES A** ; **GROUNDS RM** ; **CECCONI M.** Impact of arterial load on the agreement between pulse pressure analysis and esophageal Doppler.. *Crit Care*, 2013, vol. 17 (3), R113
- **ZHANG J** ; **CRITCHLEY LA** ; **HUANG L.** Five algorithms that calculate cardiac output from the arterial waveform: a comparison with Doppler ultrasound.. *Br J Anaesth*, 2015, vol. 115, 392-402
- **CAILLARD A** ; **GAYAT E** ; **TANTOT A** ; **DUBREUIL G** ; **M'BAKULU E** ; **MADADAKI C** ; **BART F** ; **BRESSON D** ; **FROELICH S** ; **MEBAZAA A**. Comparison of cardiac output measured by oesophageal Doppler ultrasonography or pulse pressure contour wave analysis.. *Br J Anaesth*, 2015, vol. 114, 893-900
- **ATLAS G** ; **BERGER J** ; **DHAR S.** Afterload assessment with or without central venous pressure: A preliminary clinical comparison.. *Cardiovasc Eng*, 2010, vol. 10 (4), 246-252